# EUROPEAN PATENT SPECIFICATION

(11) **EP 2 790 739 B1**
(45) Date of publication and mention of the grant of the patent: **26.09.2018**
(21) Application number: 12750728.3
(22) Date of filing: 08.08.2012
(51) Int. Cl.: A61K 49/00, A61K 47/69, A61K 49/18, A61P 35/00, A61P 9/10, A61P 9/04

(54) **NANOPARTICLES AND USES THEREOF**
NANOTEILCHEN UND IHRE ANWENDUNGEN
NANOPARTICULES ET LEURS UTILISATIONS

(30) Priority: 16.12.2011 EP 11398008
(43) Date of publication of application: 22.10.2014
(73) Proprietor: Biocant - Associação De Transferência De Tecnologia, Cantanhede 3060-197 (PT)
(72) Inventor: DA SILVA FERREIRA, Lino, 3030-076 Coimbra (PT); NEVES PIRES DAS NEVES, Ricardo, 3810-131 Aveiro (PT); GOMES, Renata, Sofia, Mota, London, SE5 9NU (GB)
(74) Representative: McNamara, Kathryn
(86) International application number: PCT/EP2012/065550
(87) International publication number: WO 2013/087234

(56) References cited:
- WO-A1-00/50050
- WO-A1-2008/107729
- WO-A1-2009/010071
- WO-A2-2011/053803
- HAN R. ET AL.: "Surface modification of poly(d,L-lactic-co-glycolic acid) nanoparticles with protamine anhanced cross-presentation of encapsulated ovalbumin by bone marrow-derived dendritic cells", JOURNAL OF BIOMEDICAL MATERIAL RESEARCH A, vol. 96, no. 1, 5 November 2010 (2010-11-05), pages 142-148, XP002678573,
- VOGEL V. ET AL.: "Oligonucleotide-protamine-albumin nanoparticles: preparation, physical properties, and intracellular distribution.", JOURNAL OF CONTROLLED RELEASE, vol. 103, 8 January 2005 (2005-01-08), pages 99-111, XP027664438,
- MAN LUNG YEUNG: "siRNA, miRNA and HIV: promises and challenges", CELL RESEARCH, vol. 15, 1 January 2005 (2005-01-01), pages 935-946, XP055011287, DOI: 10.1038/sj.cr.7290371
- HORISAWA E. ET AL.: "Size-Dependency of DL-Lactide/glycolide Copolymer Particulates for Intra-Articular Delivery System on Phagocytosis in Rat Synovium", PHARMACEUTICAL RESEARCH, vol. 19, no. 2, 1 February 2002 (2002-02-01), pages 132-139, XP002678575,
- RUIZ-CABELLO J. ET AL.: "In Vivo "Hot Spot" MR Imaging of Neural Stem Cells Using Fluorinated Nanoparticles", MAGNETIC RESONANCE IN MEDICINE, vol. 60, no. 6, 1 September 2008 (2008-09-01), pages 1506-1511, XP002678576,

## Description

### Field of the Invention

The invention relates to nanoparticles (NPs) for intracellular delivery of therapeutic agents. In particular, the invention relates to a NP conjugate comprising a NP, a cationic agent, a therapeutic agent and at least one imaging agent including a perfluorocarbon, wherein the cationic agent is protamine sulphate, and to therapeutic uses thereof.

### Background of the Invention

NPs are very promising for the intracellular delivery of anticancer and immunomodulatory drugs, stem cell differentiation biomolecules and cell activity modulators. They can be designed to target specific organs and to release their payload at specific sub-cellular regions with desired kinetics (Ferreira L et al. (2008) Cell Stem Cell 3:136-146). Although initial studies in the area of intracellular drug delivery have been performed in the delivery of deoxyribonucleic acid (DNA), there is an increasing interest in the use of other molecules to modulate cell activity.

The requirements on drug delivery systems are generally to improve efficacy and to reduce toxicity through enhancement of specificity. The application of NPs in intracellular drug delivery is advantageous over traditional delivery systems, as they have a high drug loading capacity, are stable in aqueous environments and for long-term storage, are non-toxic and non-immunogenic and may be targeted *in vivo* using antibodies or other ligands, for example.

For example, a study by Vogel et al (Journal of Controlled Release. 103 (2005) 99-111) demonstrated that nanoparticles comprising protamine, human serum albumin and phosphorothioates of oligodesoxynucleotides (ODNs) were capable of crossing cellular membranes and releasing the ODNs within the cell, and were stable in salt solution for at least several hours.

Furthermore, NPs can encapsulate or be conjugated to probes so that they can be visualised non-invasively by magnetic resonance imaging (MRI), fluorescence imaging and such like. Accordingly, they could be useful in measuring, understanding and manipulating cells, both *in vitro* and *in vivo.* Monitoring *in vivo* cell trafficking by non-invasive means is also critical to improving cellular therapeutics, drug delivery and understanding disease progression.

The use of nanoscale fluorine-based particles to track cells has previously been reported (Ahrens et al. (2005) Nat Biotechnol 23 983-987; Partlow et al. (2007) FASEB J21: 1647-1654). However, these particles were formulated as micelles or emulsions. Such micelles and emulsions are disadvantageous due to their relatively low stability, heterogeneity in size and the difficulty in achieving their functionality. Pharmacokinetics and cell penetration also represent potential obstacles to therapeutic formulations having an intracellular component. For example, the dose and bioactivity of therapeutic agents released by NPs is dependent on several variables including (i) the kinetics of cellular attachment and internalisation, (ii) the intracellular pathway and final location of the NPs and (iii) the cell cycle. The kinetics of cellular attachment and internalisation is affected by NP size, shape, charge and surface chemistry/bioactivity (Ferreira L (2009) J Cell Biochem 108: 746-752; Sahay G et al. (2010) J Control Release 145:182-195; Duncan (2011) Curr Opin Biotechnol 22(4):492-501). The mitotic index affects also the concentration of the drug that is released over time. The partitioning of NPs present in the cell during cell division is random and asymmetric and, thus, the concentration of NPs in each daughter cell after division may be different (Summers HD et al. (2011) Nat Nanotechnol 6: 170-174). Finally, the route of NP internalisation and its final location within the cell plays an important role in the delivered dose and its bioactivity. The cell has a high number of compartments and, therefore, it is important that the NP releases its content at the right place in order to have the desired magnitude and duration of effect.

So far, there is no formulation for the *in vivo* delivery of therapeutically active agents, with simultaneous control in tracking, pharmacokinetics and internalisation.

### Summary of the Invention

In a first aspect, the invention provides a nanoparticle conjugate for intracellular delivery of a therapeutic agent, wherein the conjugate comprises a nanoparticle, a cationic agent, the therapeutic agent and at least one imaging agent including a perfluorocarbon, wherein the cationic agent is protamine sulphate.

In an embodiment, the intracellular delivery is mediated by endosomes. Preferably, the nanoparticles are retained in the endolysosomal compartment.

In an embodiment, the nanoparticle is polymeric, preferably formed from poly(lactic acid-co-glycolic acid).

The perfluorocarbon is preferably perfluoro-1,5-crown ether.

In an embodiment, the therapeutic agent is a protein, peptide or oligonucleotide. Preferably, the oligonucleotide is miRNA.

Most preferably, the NP conjugate comprises poly(lactic acid-co-glycolic acid), perfluoro-1,5-crown ether, protamine sulphate and miRNA.

A method of preparing a nanoparticle conjugate according to the invention in its first aspect is also envisaged.

In a preferred embodiment, the conjugate can be used for simultaneous imaging and drug delivery.

In a second aspect, the invention provides a nanoparticle conjugate for use in a method of transfecting a mammalian cell with a therapeutic oligonucleotide, the method comprising contacting the mammalian cell with the conjugate, the conjugate comprising a nanoparticle, a cationic agent selected from chitosan and protamine sulphate, the therapeutic oligonucleotide and at least one imaging agent including a perfluorocarbon.

The mammalian cell may be an endothelial cell, a mononuclear cell, a cardiac progenitor cell or a bone marrow cell.

In an embodiment, the method is performed *in vitro.* In an alternative embodiment, the method is performed *ex vivo.* Alternatively the method is performed *in vivo.*

In a third aspect, the invention provides a mammalian cell transfected with a nanoparticle conjugate, the conjugate comprising a nanoparticle, a cationic agent, a therapeutic oligonucleotide and at least one imaging agent including a perfluorocarbon, wherein the cationic agent is protamine sulphate.

The mammalian cell may be an endothelial cell, a mononuclear cell, a cardiac progenitor cell or a bone marrow cell.

In a fourth aspect, a pharmaceutical composition comprising a nanoparticle conjugate of the first aspect and/or a mammalian cell of the third aspect is provided.

In a fifth aspect, the invention provides a kit comprising a nanoparticle conjugate of the first aspect and a mammalian cell, wherein the therapeutic agent comprised in the conjugate is an oligonucleotide and the mammalian cell is suitable for transfection with the nanoparticle conjugate.

In a sixth aspect, the invention provides a nanoparticle conjugate for use in therapy, wherein the conjugate comprises a nanoparticle, a cationic agent, a therapeutic agent and at least one imaging agent including a perfluorocarbon, wherein the cationic agent is protamine sulphate.

In a seventh aspect, the invention provides a mammalian cell, transfected with a nanoparticle conjugate, for use in therapy, the conjugate comprising a nanoparticle, a cationic agent, a therapeutic oligonucleotide and at least one imaging agent including a perfluorocarbon, wherein the cationic agent is protamine sulphate.

In embodiments, the nanoparticle conjugate of the sixth aspect and mammalian cell of the seventh aspect are for use in inducing or preventing angiogenesis, or for use in treating, ameliorating or preventing a myocardial infarction, an ischaemic disease, disorder or condition or cancer.

The nanoparticle conjugate employed in the products of the second to seventh aspects may be any nanoparticle conjugate encompassed by the first aspect.

In an eighth aspect there is provided a nanoparticle conjugate of the first aspect, a mammalian cell of the third aspect or a pharmaceutical composition of the fourth aspect for use in a method of inducing or preventing angiogenesis in a subject or in a tissue. The methods comprise administering a nanoparticle conjugate of the first aspect, a mammalian cell of the third aspect or a pharmaceutical composition of the fourth aspect to the subject or tissue.

In a ninth aspect there is provided a nanoparticle conjugate of the first aspect, a mammalian cell of the third aspect or a pharmaceutical composition of the fourth aspect for use in a method for treating, ameliorating or preventing a myocardial infarction, an ischaemic disease, disorder or condition or cancer in a subject is provided in a ninth aspect. The methods comprise comprising administering a nanoparticle conjugate of the first aspect, a mammalian cell of the third aspect or a pharmaceutical composition of the fourth aspect to the subject.

In a tenth aspect, the invention provides a delivery system for use in increasing or decreasing blood flow *in vivo* or in tissues *in vitro* or *ex vivo,* the delivery system comprising a NP conjugate of the first aspect or a mammalian cell of the third aspect.

### Brief Description of the Drawings

So that the present invention may be more fully understood, embodiments thereof will now be described with reference to the accompanying drawings in which:
Figure 1 illustrates a schematic representation of the preparation of a NP conjugate;
Figure 2 illustrates transmission electron microscopy (TEM) of NPs;
Figure 3 illustrates mass loss of poly(lactic acid-co-glycolic acid)-perfluoro-1,5-crown ether (PLGA-PPFCE) NPs over time;
Figure 4 illustrates the diameter of PLGA-PFCE NPs over time;
Figure 5 illustrates ¹⁹F nuclear magnetic resonance (NMR) spectrum of PLGA-PFCE NPs;
Figure 6A illustrates confocal laser scanning microscopy showing uptake of PLGA-PFCE formulation by human umbilical vascular endothelial cells (HUVECs);
Figure 6B illustrates confocal laser scanning microscopy showing uptake of PLGA-PFCE formulation by mononuclear cells (MNCs);
Figure 7 illustrates MRI of PLGA-PFCE NPs (8 mg), PFCE (750 µl), PLGA-PFCE-PS-labeled HUVECs (10 x 10⁶ cells) and unlabeled HUVECs (10 x 10⁶ cells) contained in separate eppendorfs;
Figure 8 illustrates fluorescence activated cell sorting (FACS) analysis of cell viability;
Figure 9 illustrates cell survival as a function of protamine sulphate (PS) concentration;
Figure 10 illustrates FACS analysis of uptake of fluorescently labeled PLGA-PFCE by HUVECs and MNCs in the presence or absence of a variety of inhibitors;
Figure 11 illustrates the labelling of HUVECs with PLGA-PFCE NPs over seven days (D0-D7), as viewed by MRI.
Figure 12 illustrates FACS analysis of cell NP labelling;
Figure 13 illustrates confocal laser scanning microscopy showing uptake of PLGA-PFCE-miRNA formulation by HUVECs;
Figure 14 illustrates a comparison of miRNA transfection efficiency between PLGA-PFCE NPs and the known transfection agent, siPORT.
Figure 15 illustrates the uptake of PLGA-PFCE-miRNA NPs by HUVECs, and their intracellular localisation.
Figure 16 illustrates siPORT delivery of miRNAs;
Figure 17 illustrates pro-survival activity of miRNA formulations;
Figure 18 illustrates vascular studies on cells transfected with PLGA-PFCE-miRNA (tube length);
Figure 19 illustrates vascular studies on cells transfected with PLGA-PFCE-miRNA (branching points);
Figure 20 illustrates (a) the downregulation of gene expression by an miRNA delivered using PLGA-PFCE NPs, and (b) a scheme of molecular delivery of miRNAs by PLGA-PFCE NPs;
Figures 21A and 21B illustrate the survival and location of endothelial cells transplanted into ischaemic hindlimbs in an animal model. Endothelial cells alone, or endothelial cells transfected with PLGA-PFCE, PLGA-PFCE-miRNA or siPORT-miRNA were used.
Figure 22 shows representative images of ischaemic hindlimbs in an animal model treated with endothelial cells alone, or endothelial cells transfected with PLGA-PFCE, PLGA-PFCE-miRNA or siPORT-miRNA.

### Detailed Description of the Invention

The invention is directed to a novel type of nanoparticle (NP) that can be used to deliver therapeutic agents into cells. In a preferred embodiment, the NPs can be used simultaneously for cell tracking and drug delivery. The NPs can be rapidly internalised by mammalian cells, including endothelial, mononuclear and cardiac progenitor cells, where they are believed to largely remain at the endosomal compartment over time.

The term "nanoparticle" as used herein is taken to mean particles having one or more dimensions at the nanometre scale, i.e. those particles having one or more dimensions of from about 1 nm to about 1000 nm. Thus, the term "nanoparticle" encompasses both ultrafine particles (of from about 1 nm to about 100 nm) and fine particles (of from about 100 nm to about 500 nm).

### 1. Preparation of NPs

Examples of NPs include polymeric NPs, liposomes, carbon nanomaterials, ceramic NPs, emulsions, metallic particles, quantum dots and others (Ferreira L et al. (2008) Cell Stem Cell 3: 136-146). The NPs may be customised in terms of size, surface charge and attachment of any targeting moieties or the like. Any type of NP may be used in the present invention; preferably, however, the NPs are polymeric NPs.

The NPs of the present invention may be formed from any suitable biocompatible materials, which may be biodegradable or non-biodegradable. Examples of suitable biodegradable materials include collagen, fibrin, chitosan, hyaluronic acid, dextran, poly(anhydrides), poly(hydroxy acids), poly(ortho esters), poly(propylfumerates), poly(caprolactones), polyamides, polyamino acids, polyacetals, biodegradable polycyanoacrylates, biodegradable polyurethanes, poly(glycerol sebacates), especially elastomeric poly(glycerol sebacates), and polysaccharides. Non-biodegradable, yet biocompatible, materials include polypyrrole, polyanilines, polythiophene, polystyrene, polyesters, non-biodegradable polyurethanes, polyureas, poly(ethylene vinyl acetate), polypropylene, polymethacrylate, polyethylene, polycarbonates, and poly(ethylene oxide). Those skilled in the art will recognise that this is not a comprehensive list of materials appropriate for the preparation of NPs, but rather an illustrative list.

In a particularly preferred embodiment, the NPs are formed from poly(lactic acid-co-glycolic acid) (PLGA). PLGA is particularly suitable for use in the present invention because it is FDA-approved for human use, easily degraded and metabolised *in vivo* and formulations comprising PLGA are stable in aqueous environments and for long-term storage.

A suitable method for preparing NPs from PLGA is by using a spontaneous emulsification solvent diffusion technique (a so-called "single emulsion" (SE)), in which a single-phase co-solvent system in which the polymer (PLGA), and any molecules to be encapsulated, are soluble. After preparation the NPs may be centrifuged and washed before freeze-drying.

Alternatively NPs may be prepared using a double emulsion (DE) technique. This method requires the components to be mixed by sonication or homogenisation (Tabata et al. (1993) Pharm Res 10: 487-496; van de Weert et al. (2000) Pharm Res 17: 1159-1167). Typically, the DE method gives rise to NPs with a more homogeneous size distribution than the SE method. However, the SE method is simpler and more convenient than the DE method.

The size and morphology of the NPs can be controlled by factors such as the molecular weight of the polymer, polymer concentration, PVA concentration, organic solvent used to solubilise PLGA, aqueous phase pH and aqueous to organic phase (w/o) volume ratio (Song et al. (2008) Eur J Pharm Biopharm 69: 445-453; Feczkó et al. (2011) Chem Eng Process 50: 846-853; Mundargi et al. (2008) J Control Release 125: 193-209).

In alternative embodiments, the NPs can be prepared by the complexation of a polycation with a polyanion. In one embodiment, NPs are formed by the complexation of polyethylenimine (a polycation) and dextran sulfate (a polyanion) (Maia et al. (2011) ACSNano 5(1): 97-106). In another embodiment, the polycations are reducible cationic polymers composed of low-molecular weight polycations crosslinked with disulphide linkages including poly(amido ethylenimine) (Jeong et al. (2007) Biomaterials 28: 1912-1917), poly(amido amine)s (Lin et al. (2007) Bioconjugate Chem 18: 138-145) and poly(disulphide amine)s (Ou et al. (2009) Biomaterials 30: 5804-5814). In another embodiment, the polyanion is hyaluronic acid.

The foregoing methods may be adapted depending upon the materials used to make the NPs, which adaptation will be within the remit of the skilled person.

### 2. Inclusion of an Imaging Agent

In a preferred embodiment, the NPs include an imaging agent. As used herein, the term "imaging agent" can mean any agent that can be tracked non-invasively using magnetic resonance imaging (MRI), ultrasound, fluorescence imaging, confocal microscopy or such like. Suitable imaging agents include, for example fluorine compounds, such as perfluorocarbon moieties (PFCs), and fluorescent labels, such as fluorescent dyes.

PFCs have been in clinical use for decades and, as such, their biocompatibility is proven. However, PFCs are largely insoluble and potentially toxic in aqueous environments. Emulsification with lipid surfactants is one known solution to this problem, but the emulsions so produced tend to be unstable and it is challenging to attach active agents, targeting moieties or such like to an emulsion. The NPs described herein are thus advantageous, as they can avoid the need to form an emulsion, yet enable a substantial amount of PFCs to be carried. The NPs may be customised in terms of the amount and type of PFC included.

Suitable PFCs for use in the present invention include perfluorooctylbromide, perfluorodecalin, perfluorohexane, perfluoro-1,8-dicholorooctane, tris(trifluoromethyl)benzene and such like. Preferably, however, perfluoro-1,5-crown ether (PFCE) is used, which can be tracked non-invasively using ¹⁹F-MRI. In a particularly preferred embodiment, the core of the NPs is formed from PLGA and PFCE. NPs can encapsulate different PFCs having different MRI signatures. Therefore, a variety of PFCs can be used in order to allow more detailed information to be obtained.

Alternatively, or additionally, the NPs may include a fluorescent label, such as a fluorescent dye. These can be tracked non-invasively using fluorescence imaging, confocal microscopy or such like. Examples of suitable fluorescent labels include fluorescein (such as fluoresceinimine or fluorescein isothiocyanate (FITC)), rhodamine, Alexa Fluor® dyes, DyLight® Fluor dyes, ATTO dyes, boron-dipyrromethene (BODIPY) dyes and such like.

It is preferred that the NPs include at least one imaging agent, as this permits the NPs to be tracked in cells *in vitro* and/or *in vivo.* For example, they may include a PFC, but no fluorescent label, or *vice versa.* Alternatively, and preferably, they may include both an imaging agent such as an ultrasound imaging agent and a fluorescent label.

The imaging agent may be included in the NPs by any suitable means including encapsulation, covalent conjugation, physical immobilisation (for example, by electrostatic attraction, hydrophobic interaction and such like), layer-by-layer (LBL) adsorption and so on. The particular method used will depend upon the particular imaging agent and the NPs selected, and such methodology would be within the remit of a skilled person.

In another embodiment, superparamagnetic iron oxide (SPIO) NPs may be used. SPIO NPs have been used as a feasible means to enhance the contrast of cellular targets in MRI. Some SPIO NPs (for example, Feridex, Endorem and Ferucarbotran) have been approved for human use by the FDA, making them particularly suitable for use in the invention. Commercially available SPIOs, such as Endorem, can be used.

In an embodiment, SPIOs can be encapsulated by a NP as described herein, as a proton magnetic imaging agent. For example, PFCs or SPIOs can be firstly adsorbed by electrostatic or hydrophobic interactions to a polycation and then further complexed with a polyanion to form a NP.

In another embodiment, PFCs or SPIOs can be encapsulated in NPs prepared in supercritical solvents. In this case, PFCs can be used as emulsifying agents and simultaneously be incorporated in the NPs (Reverchon E. (1999) J Supercrit Fluid 15: 1-21).

In another embodiment, PFCs or SPIOs can be adsorbed or encapsulated in carbon nanotubes.

In another embodiment, the NP is itself formed by a fluoropolymer (Du et al. (2008) Biomacromolecules 9(10): 2826-33).

### 3. Inclusion of a Cationic Agent

The NP conjugates of the invention include a cationic agent at, or on, the surfaces of the NPs. This is believed to enhance uptake of the NPs by the target cells. Where the NPs are to be complexed with genetic material as the therapeutic agent, the positively charged NPs are believed to interact electrostatically with the negatively charged DNA/RNA molecules, which not only facilitates complexation of the NP-therapeutic conjugate, but which may also protect the latter from enzymatic degradation and mediate cellular entry. The cationic agent is a polycationic agent, protamine sulphate (PS).

NPs, prepared as detailed above, maybe coated with the chosen cationic agent. Alternatively the cationic agent may be attached to the surface of the NPs, for example by covalent attachment. In embodiments of the invention, a NP formulation may include a mixture of coated and uncoated NPs.

Suitable methods for including cationic agents in the NPs of the invention are as follows.

In one embodiment, the carboxyl groups of a PLGA NP, for example, can be reacted with carbonyldiimidazole, an active carbonylating agent that contains two acylimidazole leaving groups. Carbonyldiimidazole reacts with carboxylic acids under non-aqueous conditions to form N-acylimidazoles of high reactivity. An active carboxylate can then react with the amine groups of PS, where this is included as a polycationic agent, to form amide bonds.

Alternatively, the carboxyl groups of a PLGA NP can be reacted with carbodiimides. Carbodiimides are used to mediate the formation of amide linkages between a carboxylate and an amine.

In another embodiment, the cationic agent can be adsorbed to the surface of the NP using layer-by-layer (LBL) techniques.

A preferred method for coating NPs with PS is as follows. NPs (1 mg/ml) and PS (1 mg/ml) may be incubated for 10 minutes under agitation, at room temperature. After the incubation period, the NPs may be dialysed (molecular weight cut-off of 50 kDa) against distilled water and freeze-dried.

In a particularly preferred embodiment, the NPs are formed using a core of PLGA and PFCE, and are coated with PS. All of these components are FDA-approved compounds for certain biomedical applications.

If SPIO NPs are used, the NPs may be coated with PS substantially as described above, but, after the incubation period, the NPs may be washed by centrifugation with distilled water in cycles of maximum centrifugation speed for 15 minutes, later re-suspended in distilled water and freeze-dried.

These methods may be adapted depending upon the materials used to make the NPs, which adaptation will be within the remit of the skilled person.

### 4. Inclusion of a Therapeutic Agent

The NPs described herein are suitable for use with any therapeutic agent. The therapeutic agent may be encapsulated by the NPs or it may be attached to a surface or surfaces thereof to form a conjugate.

In some cases, the encapsulation of the therapeutic agent is advantageous, as higher concentrations of a drug can be encapsulated than attached at the surface. Drugs can also be delivered at a sustained rate when encapsulated compared to when simply immobilised at a NP's surface.

In other cases, the immobilisation of small concentrations of a therapeutic agent at the surfaces of the NPs can be advantageous. This may be the case when (i) the drug is very active at small concentrations, such as is the case for plasmid DNA, short interference RNA (siRNA), microRNA (miRNA), growth factors etc., (ii) the drug is very sensitive to encapsulation processes, such as those requiring mixing by sonication or homogenisation, or (iii) the drug can be recognised by a cell receptor, such as a growth factor receptor etc.

Suitable methods for encapsulating therapeutic agents inside NPs are as follows.

PLGA may be dissolved in a suitable solvent and an aqueous solution of the selected therapeutic agent plus any excipients (such as serum albumin, magnesium hydroxide etc.) may be added. Such excipients are important to increase the stability of the drug during the encapsulation process (Ferreira et al. (2007) Biomaterials 28: 2706-2717). Typically, the resulting mixture is sonicated or homogenised, at a power dependent upon the ultimate size of NPs desired. This primary emulsion may then be added to an aqueous solution of PVA and homogenised for sufficient time to create a second emulsion. The NPs may then be washed by centrifugation or dialysis, and lyophilised (Nkansah et al. (2008) Biotechnol Bioeng 100(5): 1010-1019).

Another method for encapsulating therapeutic agents inside NPs is via a single emulsion (SE) method. In this case, an aqueous solution of the therapeutic agent and any excipients is mixed with a PLGA solution (in an organic solvent or a mixture of organic solvents). The resulting mixture may then be added dropwise to an aqueous solution of PVA. After a suitable number of hours, the NPs are washed by centrifugation and resuspended in water (alternatively, a dialysis process can be used). The NPs may then be lyophilised.

Another method for encapsulating therapeutic agents within NPs is using the complexation method. Typically, this method is used to encapsulate drugs in NPs that are formed by the electrostatic interaction of polycations with polyanions. The therapeutic agent may be complexed to one of the polymeric components of the NP (i.e. the polycation or the polyanion) and then the complex of drug-polymer may be further complexed with the other NP component (Maia et al. (2011) ACS Nano 5(1): 97-106).

If, however, the selected therapeutic agents are to be attached at the surface of the NPs, the NPs may suitably be immersed in a solution of the therapeutic agent and allowed to complex under appropriate incubation conditions.

For example, LBL techniques can be used to functionalise the surfaces of the NPs and complex the therapeutic agent thereto (Labouta et al. (2010) Int J Pharm 395: 236-242). LBL techniques have been used to tailor the surface of NPs and to design drug delivery systems with controlled release, modulated via layer thickness and composition. Drugs, including dexamethasone, paclitaxel, tamoxifen and siRNA, have been successfully attached to the surface of NPs using LBL techniques (Agarwal et al. (2008) J Control Release 128: 255-260; Elbakry et al. (2009) Nanoletters 9(5): 2059-2064). In addition, LBL NPs with a pH-sensitive outer stealth layer can be used for accumulation in hypoxic tumor regions (Poon et al. (2011) ACS Nano 5(6): 4284-4292).

The therapeutic agent may be a protein, peptide, chemical compound, genetic material (i.e. an oligonucleotide) or any other active molecule.

The intracellular delivery of proteins can be an alternative approach to genetic material to manipulate cellular function. Proteins have key roles in cellular processes and thus are good therapeutic agents. However, due to poor cellular permeability and proteolysis of most proteins, intracellular delivery of proteins is challenging. NPs, as described herein, may play an important role in the protection of proteins from enzymatic degradation and pH hydrolytic effect. Thus, the proteins might be delivered fully functional to integrate and complete the deficient protein machinery inside the cell.

Examples of proteinaceous therapeutic agents that may be delivered intracellularly by the NPs described herein include enzymes (such as glucose oxidase, glucose-6-phosphate dehydrogenase, hexokinase, β-galactosidase, β-glucuronidase, glucocerebrosidase, α-mannosidase, amiloglucosidase, hexoseaminidase A, peroxidase, β-D-fructofuranosidase, neuraminidase, superoxide dismutase, catalase, asparaginase, caspase, DNase, cytochrome oxidase, ATPase and dextranase, among others); peptides (such as thymosin β4, GNRH/LHRH agonists (leuprorelin, goserelin), sandostatin (somatostatin analogue), glatiramer (immunomodulator peptide), salmon calcitonin, desmopressin, bivalirudin and eptifibatide (platelet aggregation inhibitors) and enfuvirtide (HIV cell entry inhibitor), among others); and protein modulators of signaling cascades such as the Wnt pathway, Notch pathyway, TGFβ pathway, Hedgehog pathway and Hippo pathway, among others.

Alternatively, the therapeutic agent may be a small molecule, such as daunorubicin, doxorubicin, vincristine, paclitaxel, amphotericin B, morphine, dexamethasone, retinoic acid and histamine, among others. Increasing the specificity of intracellular delivery of small molecules would not only reduce side effects but also the necessary amount of drug and, consequently, costs. It is possible to predict the interaction of the particles with the cell membrane using computer simulations and such developments are beginning to shed light on the intracellular interaction partners of targeted NPs. For example, SPIO-NPs complexed with a known mitochondrial targeting peptide have been characterised after cellular internalisation by mass spectrometry in order to identify the intracellular proteins that interact with them on their way to the mitochondria (Salaklang J et al. (2008) Angew Chem Int Ed Engl 47: 7857-7860). The NPs described herein may similarly increase the specificity of intracellular delivery of small molecules. This could be advantageous, particularly for anticancer drugs and such like, where minimising the potential side effects is key.

In a preferred embodiment, the therapeutic agent may be genetic material (i.e. an oligonucleotide), such as DNA, RNA, siRNA or miRNA. Particularly preferred are complexes of NPs and miRNAs. Such molecules are referred to herein as "NP-miRNAs".

miRNAs are short, single-stranded, non-coding RNA molecules found in eukaryotic cells. Being post-transcriptional regulators of gene expression, their binding to complementary sequences on target messenger RNA (mRNA) transcripts usually results in translational repression or target degradation and gene silencing, but possibly results in positive regulation (transcriptional and translational activation) (Bartel DP (2004) Cell 116: 281-297; Bartel DP (2009) Cell 136(2): 215-233; Wahid F et al. (2010) Biochim Biophys Acta 1803: 1231-1243).

As compared to classical drugs, miRNAs can regulate many target genes and influence a whole gene network. It is believed that the human genome may encode over 1000 miRNAs, which may target about 60% of mammalian genes, and that they are abundant in many human cell types (Bentwich I et al. (2005) Nat. Genet. 37(7): 766-70; Lewis BP et al. (2005) Cell 120(1): 15-20; Friedman RC et al. (2009) Genome Res. 19(1): 92-105; Lim LP et al. (2003) Genes Dev. 17(8): 991-1008). miRNAs are thus likely to be involved in many, if not most, biological processes.

Aberrant expression of miRNAs has been implicated in numerous disease states, and miRNA-based therapies are under investigation (Trang P et al. (2008) Oncogene 27 Suppl 2: S52-S57; Li C et al. (2009) AAPS J 11 (4): 747-757; Fasanaro P et al. (2010) Pharmacol Therapeut 125 (1): 92-104). Recent studies have begun to unveil powerful therapeutic roles for miRNA in numerous forms of ischaemic diseases (Fasanaro P et al. (2010) Pharmacol Therapeut 125(1): 92-104). As an example, miR-210 has been identified as a key player of endothelial cell response to low oxygen tension (Fasanaro P et al. (2008) J Biol Chem 283: 15878-15883), regulating mitochondrial free radical response to hypoxia (Favaro E et al. (2010) PLoS One 5: e10345). It is also a cell pro-survival factor, upregulating several angiogenic factors, inhibiting caspase activity and preventing cell apoptosis (Hu S et al. (2010) Circulation 122: S124-131). miR-132 has been reported to induce endothelial cell proliferation and endothelial tube formation in a three-dimensional collagen matrix (Anand A. et al. (2010) Nat Med 16(8): 909-914). miR-424 stabilises hypoxia-inducible factor 1α and plays an important physiological role in post-ischaemic vascular remodeling and angiogenesis (Ghosh G et al. (2010) J Clin Invest 120: 4141-4154). AmiR-92a induces angiogenesis *in vitro* and *in vivo* by regulating the expression of the integrin subunit α5 (Bonauer A et al. (2009) Science 324: 1710-1713).

However, pharmacokinetics and cell penetration represent potential obstacles to therapeutic miRNA manipulation strategies using miRNA formulations. Several strategies have been reported for the *in vivo* delivery of miRNAs, including (i) chemical modification, (ii) liposomes, and (iii) adeno-associated virus or lentivirus (Shi MA et al. (2010) Sci Signal 3: pe40; Kota J et al. (2009) Cell 137: 1005-1017). Nevertheless, so far, there is no formulation for the *in vivo* delivery of miRNA, with simultaneous control in tracking, pharmacokinetics and internalisation.

The NPs described herein are believed to be particularly efficacious in this regard. As demonstrated herein, the NPs can mediate the intracellular delivery of therapeutically useful miRNAs, notably miR-424, miR-132 and antagomir92a (AmiR-92a). As demonstrated herein, such miRNAs can exert a pro-survival effect in cells exposed to hypoxia, both *in vitro* and *in vivo.* In one embodiment, therefore, the miRNA is an miRNA that can enhance cell survival in ischaemic conditions, such as miR21, miR24 or miR221 (Hu S et al. (2011) Circulation 124: S27-34), miR125b (Ooi A. et al. (2010) Proc Natl Acad Sci USA 107: 21505-21510) or miR210 (Hu S et al. (2010) Circulation 122: S124-131). In one embodiment, the miRNA is a pro-angiogenic miRNA, such as AmiR-92a (Bonauer A et al. (2009) Science 324: 1710-1713) or miRi26 (Nicoli S et al. (2010) Nature 467: 356). Such NPs offer high efficiency in the regulatory mechanism of miRNAs and the possibility to monitor transplanted cells in ischaemic tissues by the use of non-invasive imaging techniques such as MRI, as demonstrated herein.

Furthermore, studies for miRNA delivery have traditionally explored nanoformulations that accumulate in the cytoplasm and not in the endolysosomal compartment (Shi S et al. (2012) Pharm Res 29: 97-109; Cheng C et al. (2012) Mol Pharm 9: 1481-1488). It was believed that RNA regulation occurs largely in cytoplasmic, membrane-free cellular regions (Gibbings et al. (2010) Trends Cell Biol 20: 491-501). However, recent data indicate that RNA regulation occurs in the endolysosomal compartments (Gibbings et al. (2010) Trends Cell Biol 20: 491-501; Gibbings et al. (2009) Nat Cell Biol 11: 1143-1149). Many RNA processes may be restricted spatially in the endolysosomal compartments to promote specificity and kinetic efficiency. Unfortunately, so far, no NP formulation has been developed to accumulate in the endolysosomal compartment and release the miRNA. However, the NPs described herein are believed to be particularly efficacious in this regard. As demonstrated herein (as proof of concept), endothelial and mononuclear cells rapidly internalise the NPs, which largely remain at the endolysosomal compartment over time.

The NPs described herein are also believed to be beneficial for use with other types of genetic material. Genes can be delivered by viral vectors (including retroviruses, lentiviruses and adenoviruses), non-viral vectors (including polymers and lipids) or physical delivery methods such as electroporation and nucleofaction. However, these are not without their disadvantages. Viral vectors have a risk of toxicity, immunogenicity, insertional mutagenesis and high manufacturing costs, for example. The NPs described herein are believed to be free from such disadvantages.

miRNAs for use with NPs as described herein may be natural or synthetically produced. Suitable methods will be known to those skilled in the art. For example, miR-424 (Ambion), miR-132 (Ambion) and AmiR-92a (Exiqon) may be obtained commercially, as may miR-210, -21, -24, -221, -125b, -210 and -126. All miRNAs, at any stage of development, can be used in the described NPs; thus, pre-miRNA or mature miRNA may be used, for example.

NPs as described here in may be complexed with miRNAs by incubating the NPs with the miRNAs for a suitable time and at a suitable temperature. For example, in one preferred embodiment, NP-PFCEs may be weighed, sterilised under ultraviolet (UV) light for 30 min, and resuspended in EGM-2 serum-free medium to yield a final concentration of 500 µg/ml. The suspension of NPs may then be dispersed by ultrasound (2 × 10 s; BRANSON 2510) and miRNAs may be added (at a concentration of 100 or 200 nM, for example) and allowed to complex to the NPs for 1 h at 37 °C, with intermittent agitation.

Such methods may be adapted depending upon the materials used to make the NPs and the chosen therapeutic agent, which adaptation will be within the remit of the skilled person.

### 5. Inclusion of a Cell Trafficking Agent

Therapeutic agents act at different cellular organelles. For example, DNA is processed at the cell nucleus, siRNA at the cytoplasm or cellular organelles and small inhibitors for the treatment of lysosomal storage disorders at the lysosome. Drug-containing NPs may therefore need to be directed to precise locations within the cell, in order to lead to the desired magnitude and duration of the drug effects.

Most of the current NP formulations are not designed to target specific organelles in the cell. However, NPs can be decorated with ligands or organelle-specific targeting moieties to deliver their content at specific organelles; for example, Tat peptide can be used to target the nucleus (de la Fuente JM (2005) Bioconjug Chem 16: 1176-1180), mitochondrial structural protein (MSP) can be used to target the mitochondria (Hoshino A et al. (2004) Microbiol Immunol 48: 985-994) and an antigenic peptide modified with an endoplasmic reticulum (ER)-insertional sequence can be used to target the ER (Matsuo K et al. (2007) Biochem Biophys Res Commun 362: 1069-1072).

The NP conjugates described herein may thus be adorned with a ligand or a cell trafficking agent, such as a nuclear localisation signal, a mitochondrial localisation signal, an ER signal peptide, an ER retrieval sequence or such like, as is described in the art.

An alternative strategy, however, is to avoid release of the NPs from the endosomal compartment and to use the endomembrane intracellular trafficking system. In other embodiments, therefore, a trafficking agent is not included in the NPs as described herein. In this regard, recent data have indicated that exosomes, which are vesicles released from the endolysosomal pathway, contain RNA and miRNAs; for example, approximately 121 miRNAs have been found in exosomes (Valadi et al. (2007) Nat Cell Biol 9(6): 654-659). Furthermore, recent studies have indicated that these exosome-like vesicles contain single-stranded, mature miRNAs in addition to GW182 and AGO2, which are the two main components of the RNA-induced silencing complex (RISC) (Gibbings et al. (2009) Nat Cell Biol 11(9): 1143-1149). Therefore, the NP formulations described herein offer clear advantages over other formulations (e.g. siPORT transfection agent (Ambion)), as they may be retained in the endolysosomal compartment. Thus, in an embodiment, the NPs described herein are not released into the cell cytoplasm following their delivery into cells.

In summary, therefore a NP conjugate can be prepared by coating or attaching the cationic agent protamine sulphate to the surface of a suitable NP, and incorporating a therapeutic agent into the conjugate. The therapeutic agent may be incorporated by any suitable means. It may be complexed to the NP, for example by complexation with the cationic agent, or it may be encapsulated by the NP. The NP conjugate also includes at least one imaging agent including a perfluorocarbon. For example, an imaging agent maybe encapsulated in the NP, or maybe attached to a surface thereof.

### 6. Characterisation of NPs

As already mentioned, the kinetics of cellular attachment and internalisation is affected by NP size, shape, charge and surface chemistry/bioactivity.

NPs for use as described herein have one or more dimensions of from about 1 nm to about 1000 nm, and preferably from about 1 nm to about 500 nm. The NPs maybe fine particles, that is to say, they may have one or more dimensions of from about 100 nm to about 500 nm. For example, the NPs may have an average diameter of about 100-250, 200-350, 300-450, 250-500, 400-500 or 200-400 nm. The NPs maybe ultrafine particles, that is to say, they may have one or more dimensions of from about 1 nm to about 100 nm. For example, the NPs may have an average diameter of about 1-25, 25-50, 50-75, 75-100, 20-40, 40-60 or 60-80 nm. The NPs maybe a mixture of fine and ultrafine particles. For example, the NPs may have an average diameter of about 25-125, 50-150, 75-200, 50-250 or 75-400 nm.

NPs for use as described herein may be of any shape and surface morphology. For example, the NPs maybe substantially spherical. The surface maybe smooth or fissured. The surface may comprise subnanometric striations of alternating anionic and hydrophobic groups (Verma et al. (2008) Nat Mater 7: 588-595).

The NPs described herein have a positive surface charge, owing to their inclusion of the polycationic agent protamine sulfate. Positive surface charge is believed to be beneficial, as it may promote interaction of the NPs at target cell membranes. In addition, the positive surface charge advantageously facilitates complexation to negatively charged moieties, such as miRNA.

The NPs may be characterised in terms of particle size, shape, morphology, surface charge and such like using standard methods in the art.

For example, particle size may be determined using light scattering, using a Zeta PALS Zeta Potential Analyzer and ZetaPlus Particle Sizing Software, v. 2.27 (Brookhaven Instruments Corporation), for example. In one such embodiment, NPs may be suspended in 1 mM potassium chloride buffer at pH 5.5 (500 µg/ml) and sonicated for short periods (<1 min). Typically, all sizing measurements are performed at 25 °C, and all data are recorded at 90°, with an equilibration time of 5 min and individual run times of 120 s (five runs per measurement).

Particle shape, morphology and size can be evaluated using TEM (JEOL JEM-100 SX microscope at 80 kV) following negative staining with osmium tetroxide, for example. In one such embodiment, a NP suspension (5 µl, 5 mg/ml in water) and osmium tetroxide solution (5 µl, 5% w/v) may suitably be placed on a 400 mesh copper grid with a carbon-coated Formvar membrane. The sample can be dried overnight before examination by TEM.

Other methods for characterising NPs as described herein will be known to the skilled person.

### 7. Cell Uptake of NPs

The problem with many known drug delivery systems is that they are recognised by the human body as foreign and, hence, they become easily opsonised and removed from the circulation long before they have fulfilled their function. The NPs described herein are thus advantageous, as they are able to achieve a better effect of targeted drug delivery systems due to their rapid internalisation by target cells.

Understanding the mechanism of internalisation is critical for the efficient labeling of cells with NPs and the delivery of biomolecules. Endocytosis may occur through several distinct mechanisms including phagocytosis, a process restricted to specialised mammalian cells, and pinocytosis, which occurs in all mammalian cells and encompasses macropinocytosis, clathrin-mediated endocytosis, caveolae-mediated endocytosis, as well as other less characterised clathrin- and caveolae-independent endocytic pathways (Ferreira et al. (2008) Cell Stem Cell 3: 136-146; Conner et al. (2003) Nature 422: 3744; Lu et al. (2007) Nano Letters 7: 149-154). Internalisation under endocytosis-inhibiting conditions has also been documented (Kostarelos et al. (2007) Nat Nanotechnol 2: 108-113).

It is believed that the NPs described herein may be internalised by the target cells by receptor-mediated endocytosis or some form of active transport. Alternatively, they may enter the cell via a non-specific internalisation process. The cationic agent at, or on the surface of, the NPs is believed to assist in the interaction of the NPs with their target cells, for example, at the appropriate receptor, membrane channel, gate or such like. If a negatively-charged therapeutic agent, such as miRNA, is present at the surfaces of the NPs, this may further assist in the cell surface interaction. For example, the miRNA molecules may themselves target the NPs to their corresponding cell surface receptors.

Once the target cells have internalised the NPs, the latter are believed to be taken up in endosomes. Any early endosomes formed may develop and fuse to form late endosomes.

In the absence of inclusion of an intracellular trafficking agent, the NPs are believed to largely remain at the endosomal compartment over time. Where miRNAs are used as the therapeutic agent, the NP-miRNA complexes may thus remain inside the endosomes. Staying inside endosomes may be advantageous, as it may contribute to more close encounters with potential partner molecules. In this regard, the endomembrane system seems to have a specific way of assessing vesicles and sorting cargoes according to cellular needs. The NPs described herein may thus ride the endomembrane system and be transported to their final destination in a more direct and effective way.

Alternatively, the miRNA molecules may be released from the NP-miRNAs into the cell cytoplasm. This is particularly the case where a trafficking agent is included in the NPs as described herein.

Any cell type may be targeted by the NPs described herein. In a preferred embodiment, the target cells are mononuclear cells (MNCs) or endothelial cells (particularly human umbilical vascular endothelial cells (HUVECs)). In alternative embodiments, the target cells are cardiac progenitor cells, bone marrow cells or cancer (tumour) cells.

Cell uptake of NPs may be achieved *in vitro* as follows.

MNCs, for example, may be obtained from umbilical cord blood (UCB) samples using Ficoll density gradient separation. HUVECs, for example, may be isolated from human umbilical vein using standard procedures in the art. It will be known to the skilled person how to obtain cardiac progenitor cells, bone marrow cells and cancer cells.

MNCs, HUVECs or other target cells may be labeled with NPs as herein described using any suitable method. For example, 2 x 10⁶ cells/condition may be labeled with NPs in an amount of between 500 µg/ml and 8 mg/ml for 4 h in serum-free EGM-2 (HUVECs) or serum-free M199 (MNCs). This may be performed in any suitable cell culture vessel.

After incubation, the cells (for example, HUVECs) may be washed, suitably three times, with phosphate buffered saline (PBS) and trypsinised (with 0.2% (v/v) trypsin, for example), to release them from the walls of the culture vessel. MNCs may be washed, suitably three times, with PBS. The cells may be passed through a suitable separating column, such as a MACS column (Miltenyi Biotec), to further separate them from any non-internalised NPs. Cell viability may be determined using trypan blue exclusion, as is standard in the art. The cells, once labeled with NPs in this way, may be resuspended in PBS, frozen at -80 °C and/or freeze-dried, as desired.

If cells are to be transfected (i.e. a NP as described herein is to deliver genetic material, such as miRNA, into the target cells), the target cells (such as HUVECs) may be washed twice with pre-warmed PBS and the suspension of NP-miRNA (or other genetic material) may be added to the cells for incubation at 37 °C for 4 h. Cells are suitably transfected keeping a ratio of 1.25 mg of NP-miRNA (or other genetic material) per million HUVECs.

In a SiPORT transfection procedure, a suspension of the SiPORT transfection agent and the genetic material (for example, miRNAs) may be dispensed onto PBS-pre-washed cells and incubated for 4 h at 37 °C.

Alternatively, the cells can be seeded onto circular 20 mm glass coverslips coated with 0.2% gelatin inside a 24 well plate and left to adhere. The cells may then be incubated with NPs (500 µg/ml) for 4 h. Once the incubations are terminated using a standard procedure in the art, the coverslips may be washed gently, optionally staining for lysosomes (using LysoTracker red DND-99 at 50 nM, 20 min incubation) or mitochondrial activity (using MitoTracker Red CM-Ros, 50 nM, 15 min incubation) may be carried out and the cells may be rewashed as necessary.

In another embodiment, MNCs (1×10⁶ cells) or HUVECs (0.2×10⁶ cells), for example, may be incubated for 4 h in serum free M199 or serum free EGM-2, respectively, containing variable concentration of NPs in a 24 well plate. After 4 h, the HUVECs may be washed with PBS on the plate three times. The cells may then be re-suspended in serum free EGM-2, prior to trypsinisation with 0.2% (w/v) trypsin solution and centrifugation at 1000 rpm for 5 min.

Cell uptake of NPs may be achieved in cells for transplantation in human patients. In human melanoma patients, dendritic cells (15 x 10⁶ cells in 200 ml) labeled with NP-PFCE using methods described herein can be transplanted directly into a lymph node region. Suitable transplantation methods are described elsewhere (de Vries et al. (2005) Nat Biotechnol 23(11): 1407-1413).

As a further example, and as described in more detail in the Examples section, NPs complexed to pro-angiogenic miRNAs may be transfected *in vitro* or *ex vivo* into suitable cells, such as mononuclear, endothelial, cardiac progenitor or bone marrow cells, and the transfected cells injected into ischaemic muscle tissue in a mammalian recipient. The miRNAs are delivered to the endolysosomal compartment by the NPs, where they are able to complex with a target gene. As demonstrated herein, complexation with the target gene causes a change in gene expression, which results in increased blood perfusion and, hence, the treatment of ischaemic disease.

Alternatively, anti-angiogenic miRNAs could be used, to target cancer cells.

Thus, also provided herein is a mammalian cell transfected with a NP conjugate, the conjugate comprising a NP, a cationic agent and a therapeutic oligonucleotide. The invention as described herein also encompasses a kit comprising such a NP conjugate and a mammalian cell. The kit may further comprise instructions for transfecting the mammalian cell with the NP conjugate.

The NP conjugate will be as hereinbefore described. The mammalian cell can be any cell that is suitable for transfection. The term "suitable for", in this instance, can mean a cell that is ready for transfection or a cell that can be treated so as to be ready for transfection, as would be known to the skilled person. The mammalian cell is preferably an endothelial cell, an MNC, a cardiac progenitor cell or a bone marrow cell, but can be any cell type. The therapeutic oligonucleotide can be any type of genetic material as hereinbefore described. Preferably, however, the therapeutic oligonucleotide is miRNA. Suitable methods for transfecting mammalian cells with NP conjugates as described herein are provided in the Examples. Such methods could be detailed in the instructions that are optionally included in the kits of the invention.

The foregoing methods may be adapted depending upon the particular NPs, therapeutic agents and target cells selected, which adaptation will be within the remit of the skilled person.

Cell uptake of NPs may be achieved *in vivo* as follows.

NPs containing the chosen therapeutic agent can be injected into the brain using stereotaxic surgery. After anaesthesia, an incision into the dorsal aspect of the head can be made, exposing the cranium and the bregma, and a fine dental air-drill can be used to drill a hole in the skull. Slow microinjection equipment can be used to deliver NPs (2-10 µl in mice; 500 µg/ml of NPs containing the therapeutic agent) into the substantia nigra or the brain left ventricle (Bharali et al. (2005) PNAS 102: 11539-11544).

In another embodiment, NPs containing the therapeutic agent can be injected in the bloodstream to target tumours. In this case, NPs can cross endothelial cells and accumulate within tumour cells, a process referred to as enhanced permeation and retention (EPR) (Farokhzad et al. (2006) PNAS 103: 6315-6320).

In another embodiment, NPs containing the therapeutic agent can be injected in the bloodstream to target ischaemic areas. If active targeting is desired, a molecular target must be identified at the desired site of drug action that is substantially enriched relative to the rest of the body, and a ligand to that target must be provided. For example, NPs coated with angiotensin II type 1 (100 µl in mice) may be injected into the right jugular vein, in order to target the infarcted heart (Dvir et al. (2011) Nano Lett 11: 4411-4414).

Again, the foregoing methods may be adapted depending upon the particular NPs, therapeutic agents and target cells selected, which adaptation will be within the remit of the skilled person.

### 8. In Vitro Cell Tracking

*In vitro* cell tracking is useful for measuring, understanding and manipulating cells, evaluating cellular therapeutics and drug delivery and understanding disease progression.

NP-labeled cells may be tracked *in vitro* using any suitable technique. In a preferred embodiment, the NPs may comprise a fluorine compound that can be tracked by ¹⁹F-MRI, for example.

The inclusion of a fluorine compound, such as PFCE, may be performed as described herein. The target cells may be labeled with the fluorine-containing NPs (for example, NP-PFCEs) and lyophilised, also as described herein. The lyophilised cells may then be characterised by ¹⁹F NMR according to standard procedures in the art. For example, ¹⁹F NMR analyses may be performed in a 600 MHz NMR Varian Instrument. Suitably, NPs (5 mg) are dissolved in a suitable volume of ¹H-dichloromethane (such as 300 µl) and transferred to a 5 mm NMR tube. A sealed 3 mm tube containing trifluoroacetic acid with ¹H-dichloromethane may be inserted in the 5 mm tube and used as an internal reference. A total of 50 scans may be run, with pulses at 90° after a relaxation time of 15 seconds.

Alternatively, confocal microscopy may be used. In this embodiment, target cells may be seeded onto glass coverslips, incubated with 500 µg/ml fluorine-containing NPs (for example, NP-PFCEs), washed gently and, optionally, life stained for lysosomes or mitochondrial activity, as described herein, and rewashed prior to fixation. The cells may then be fixed with 4% (w/v, in water) paraformaldehyde (EMS, Hatfield, USA) for 10 min at room temperature and washed with PBS.

For certain conditions, the slides may be mounted straight away with mounting medium-4',6-diamidino-2-phenylindole (DAPI; Sigma-Aldrich) on a glass slide.

However, in some conditions, cell membrane staining may be performed. In these embodiments, mouse antibodies against human CD31 (Dako, Glostrup, Denmark) or CD45 (BD Biosciences, Spain) may be used to stain the membrane of target cells such as HUVECs or cord blood MNCs. Cells may be washed three times after fixation (MNCs may be washed by centrifugation), the cells may then be blocked with 2% bovine serum albumin (BSA) (Sigma-Aldrich) in PBS for 30 min at room temperature. The CD31/CD45 (both at 1:50 dilution) primary antibody may be added in PBS for 1 h at room temperature followed by 3 washes in PBS. The binding of primary antibodies may be detected with anti-mouse DyLight 649 conjugate (at a dilution of 1:200) (Jackson ImmunoResearch, USA). The nucleus of cells may be stained with mounting medium-DAPI. After the indirect labelling, the cells may be examined with a Zeiss Laser Scanning Microscope Meta 510 or such like.

In another preferred embodiment, fluorescence activated cell sorting (FACS) analysis may be used. Target cells, such as MNCs or HUVECs, may be labeled with NPs in a 24-well plate and later washed with PBS, as described herein. The cells may be re-suspended in serum free EGM-2 with mitotracker red CMX-ROS at 50 nM for 15 min at 37 °C in a carbon dioxide incubator. The cells may then be trypsinised with 0.2% (w/v) trypsin solution, centrifuged at 1000 rpm for 5 min, and fixed with 4% paraformaldehyde for 10 min at room temperature. After fixation, the cells may be rewashed and then re-suspended in 500 µl of PBS, ready for FACS analysis. A total of 80,000 events may be recorded per measurement.

MNCs may be transferred to a 2 ml eppendorf and centrifuged for 15 min at 20 °C, 1300 rcfs, then the MNCs may be further washed by using anti-CD45 human microbeads via a Mini MACS system and protocol (Miltenyi Biotec, UK) so as to ensure that only MNCs, and no free NPs, are present in the final solution. The cells may then be incubated with mitotracker (50 nM) for 15 min at 37 °C, washed once with PBS and fixed with 4% paraformaldehyde for 10 min at room temperature. After fixation, the cells may be washed and resuspended in 500 µl of PBS before FACS analysis. A total of 80,000 events may be recorded per measurement.

If the target cells have been transfected, for example, using a SiPORT transfection procedure as described herein, transfection visualisation may be performed under a fluorescence microscope using NP-PFCEs labeled with fluorescein and miRIDIAN microRNA mimic transfection control with Dy547 (Dharmacon).

In another embodiment, fluoresence imaging may be used. For example, sample tubes may be placed inside a FluorVivo 300 (INDEC Biosystems, Santa Clara, USA) or such like. Exposure times are typically between 0.05 and 0.15 s.

Any of the foregoing methods may be adapted depending upon the particular NPs, imaging agents and target cells selected, which adaptation will be within the remit of the skilled person.

### 9. In Vivo Cell Tracking

NPs may also be tracked *in vivo,* by any suitable means, preferably non-invasively. This could be useful in measuring, understanding and manipulating cells *in vivo.* Monitoring *in vivo* cell trafficking by non-invasive means is also critical to improving cellular therapeutics, drug delivery and understanding disease progression. It may be used to optimise the route of delivery, cell localisation, dosage and other factors.

MRI may suitably be used for this purpose, owing to its non-invasive nature, intrinsic anatomic contrast, high resolution and because it does not use ionising radiation. ¹⁹F-MRI, in particular, allows extremely specific detection and quantification of cell numbers directly from *in vivo* image data and, thus, is a preferred procedure. Such *in vivo* imaging enables the localisation, quantity and viability of cellular therapeutics to be carefully monitored.

*In vivo* tracking may be performed in animal models of diseases such as myocardial infarction and hindlimb injury, for example. By way of illustration, NPs as described herein can be used to track cardiosphere derived cells (CDCs; also known as cardiac stem cells) and bone marrow-derived cells into rat models of myocardial infarction, as follows.

Rat CDCs may be isolated from hearts of Sprague Dawley rats (Harlan, UK) and cultured as described in Carr et al. (2011) PlosOne 6: 10. CDCs (1x10⁷ cells) at passage 2 may then be labeled with 500 µg/ml of NPs per 400,000 cells for 4 h at 37 °C and 5% CO₂ in explant medium (EM) comprising Iscove's modified Dulbecco's medium (IMDM; Invitrogen) supplemented with 2% foetal bovine serum (FBS; Invitrogen), 1 U/ml penicillin, 1 µg/ml streptomycin and 0.2 mM L-glutamine (Gibco). After the incubation period, cells may be washed three times with PBS and trypsinised. The cells may be re-suspended into 50 µl PBS (Sigma) and placed into U-100 insulin syringes (Becton, Dickinson) maintained in ice until required for injection.

Rat bone marrow derived cells may be isolated from the femur and tibia of sacrificed Sprague Dawley rats (Harlan, UK). The ends of the bones may be cut off and the cavity flushed with PBS to remove whole bone marrow. Total mononuclear bone marrow (MBM) derived cells may be isolated via density gradient centrifugation using Ficoll-Paque Premium (GE Healthcare, UK). MBMs (1.2x10⁷ cells) straight after isolation may be labeled with 500 µg/ml NPs per 1,000,000 cells for 4 h at 37 °C and 5% CO₂ in medium199 (M199) supplemented with 2% FBS (Invitrogen), 1 U/ml penicillin and 1 µg/ml streptomycin (Gibco). After the incubation period, cells may be washed three times with PBS. The cells may be re-suspended into 50 µl PBS (Sigma) and placed into U-100 insulin syringes (Becton, Dickinson & Company) maintained in ice until required for injection.

The left anterior descending (LAD) coronary artery of Sprague Dawley rats (Harlan, UK) may then be occluded using a method described elsewhere (Michael et al. (1995) Am J Physiol 269: H2147-2154). In brief, following anaesthesia, using 2% isoflurane in oxygen, and thoracotamy, the pericardium may be removed and a 5-0 prolene suture placed under the LAD, about 2 mm from the origin. The suture may be tied around a small piece of polyethylene tubing, occluding the LAD, and the chest closed. After 50 minutes, the chest may be re-opened and the tubing removed to allow reperfusion. Ten minutes after ischaemia/reperfusion, cells labeled with NPs with or without miRNAs may be injected over four sites in the peri-infarct region. In sham animals, thoracotamy may be performed but no stitch is placed in the heart. Animals should be allowed free access to standard rodent chow and water throughout the study.

Cell tracking may be measured via ¹⁹F MRI and cardiac function may be measured using ¹H cardiac gated CINE MRI on a horizontal bore 7 Tesla, 300MhZ Varian instrument using VnmrJ software.

As a further illustration, NPs as described herein can be used to track endothelial cells and smooth muscle cells into mice and rabbit hindlimb models, as follows.

A model of acute ischaemia may be induced in the right hindlimb of rabbits and mice by surgical procedures, as previously described in Prompers et al. (2006) NMR Biomed 19:927-953. Briefly, the superficial part of the femoral artery may be ligated under ketamine (Ketalar, Pfizer 0.3 ml/kg) and medetomidine (Domitor, Orion, 0.3 ml/kg) anaesthesia. The re-entry branches for the collaterals growing from the lateral circumflex and deep femoral arteries may be ligated. In this model, the calf region is ischaemic while the thigh remains normoperfused. In a sham operation, all other procedures are similar to the actual operation except that ligatures may be placed loosely around vessels, not blocking blood flow. The rabbits and mice may receive cells labeled with NPs with or without miRNAs, or cells without NPs at all, via intramuscular injection. Intramuscular injection may be performed in the semimembranosus, abductor cruris cranialis, quadriceps femoris, tibialis anterior and gastrocnemius muscles using a 1 ml syringe and a 25-gauge needle under ketamine- medetomidine anaesthesia.

Cell tracking may be measured via ¹⁹F MRI, flow measured using VEVO ultrasound and cellular proliferation using IVIS systems. Suitable methods are disclosed in the Examples.

Such methods may be adapted depending upon the particular NPs, imaging agents and target cells selected, and the subject to be imaged, which adaptation will be within the remit of the skilled person.

Alternatively, *in vivo* fluorescence imaging may be used. For example, animal models may be placed inside a FluorVivo 300 (INDEC Biosystems, Santa Clara, USA) or such like. Exposure times are typically between 0.05 and 0.15 s.

Other techniques for use with NPs as described herein include positron emission tomography (PET), ultrasound and computed tomography (CT), for example.

Naturally, any of the foregoing methods may be adapted for use with human subjects.

### 10. Clinical Uses

Many clinical applications of the NPs described herein are envisaged. For example, MNCs, HUVECs, cardiac progenitor cells or bone marrow cells labeled with NP-miRNAs as described herein could be administered to patients suffering a myocardial infarction or other ischaemic disease, disorder or condition. It is thought that cancer patients may also benefit from treatment with NP-miRNAs as described herein, as a subset of the latter are believed to be anti-angiogenic.

The invention thus provides pharmaceutical compositions and delivery systems comprising a NP conjugate and/or a mammalian cell transfected with a NP conjugate, as described herein. The invention provides first and further medical uses of the NP conjugates and transfected cells. Hence, in certain aspects, there is provided a NP conjugate or a transfected cell for use in therapy or for use in treating the specific diseases, disorders and conditions identified herein.

The terms "myocardial infarction" and "ischaemia" will be known to the skilled person. Myocardial infarction, also known as a heart attack, results from the interruption of blood supply to a part of the heart, causing heart cells to die. This is most commonly due to blockage of a coronary artery following the rupture of a vulnerable atherosclerotic plaque. The resulting ischaemia (i.e. restriction in blood supply) and ensuing oxygen shortage, if left untreated for a sufficient period of time, can cause damage or death of heart muscle tissue.

Ischaemic diseases, disorders and conditions thus include myocardial infarction and ischaemic heart disease, as well as intestinal ischaemic disorders, mesenteric ischaemia, ischaemic colitis, bowel ischaemia, cerebrovascular disorders, cerebral ischaemia, stroke, ocular ischaemic syndrome, ischaemic neuropathy, blood vessel ischaemic disease, acute arterial ischaemic disorder, peripheral arterial disease, critical limb ischaemia and such like.

As demonstrated herein, the NPs described herein can be used to deliver suitable therapeutic agents to the ischaemic or infarcted heart, or to other ischaemic tissues, such as ischaemic muscle tissue or ischaemic cerebral tissue. Thus, in one embodiment, NP conjugates or transfected mammalian cells as described herein are used to treat, ameliorate or prevent a myocardial infarction or other ischaemic disease, disorder or condition. Any of the described NP conjugates may be used in accordance with these embodiments of the invention.

Underlying such therapeutic applications is the ability of the NP conjugates or transfected mammalian cells described herein to induce angiogenesis in a subject or in a tissue, as demonstrated in the Examples. Angiogenesis can lead to increased blood flow compared to the level of blood flow in the absence of such conjugates or cells, for example basal blood flow levels. Blood flow may be increased by at least 1%, 5%, 10%, 25%, 50%, 75%, 100% or more. The NP conjugates and cells may therefore be used to increase blood flow *in vivo* or in tissues *in vitro* or *ex vivo.*

There is also provided a delivery system for use in increasing blood flow *in vivo* or in tissues *in vitro* or *ex vivo,* the delivery system comprising a NP conjugate, or a mammalian cell transfected with such a NP conjugate, as described herein. In an embodiment, the delivery system is suitable for treating, ameliorating or preventing a myocardial infarction or other ischaemic disease, disorder or condition.

In another aspect of the invention, the NP conjugates or transfected mammalian cells can be used to prevent angiogenesis in a subject or in a tissue. The prevention of angiogenesis can lead to decreased blood flow compared to the level of blood flow in the absence of such conjugates or cells, for example basal blood flow levels. Blood flow may be decreased by at least 1%, 5%, 10%, 25%, 50%, 75% or more. The NP conjugates and cells may therefore be used to decrease blood flow *in vivo* or in tissues *in vitro* or *ex vivo.*

One application of such technology is in the treatment of cancer. Tumour cells need a supply of oxygen and nutrients in order to grow. As a tumour grows, it sends out signals to nearby blood vessels, causing new blood vessels to sprout towards the tumour (angiogenesis). The new blood vessels grow into the tumour, thereby providing a route for the cancer cells to spread around the body. The use of NP conjugates or transfected mammalian cells, as described herein, to prevent angiogenesis in a subject or in a tissue can therefore reduce or prevent tumour growth, i.e. the NP conjugates and cells can be used to treat, ameliorate or even prevent cancer.

There is also provided a delivery system for use in decreasing blood flow *in vivo* or in tissues *in vitro* or *ex vivo,* the delivery system comprising a NP conjugate, or a mammalian cell transfected with such a NP conjugate, as described herein. In an embodiment, the delivery system is suitable for treating, ameliorating or preventing cancer.

The term "cancer" will be known to the skilled person. Medically the term means a malignant neoplasm, and it encompasses a broad group of various diseases, all involving unregulated cell growth. There are over 200 different known cancers that afflict humans. The NP conjugates and transfected mammalian cells described herein can be used to treat, ameliorate or prevent any type of cancer, including, but not limited to basal cell carcinoma, biliary tract cancer, bladder cancer, bone cancer, brain and CNS cancer, breast cancer, cervical cancer, choriocarcinoma, colon cancer, rectum cancer, connective tissue cancer, cancer of the digestive system, endometrial cancer, oesophageal cancer, eye cancer, cancer of the head and/or neck, gastric cancer, intra-epithelial neoplasm, kidney cancer, larynx cancer, leukemia, liver cancer, lung cancer (e. g. small cell and non-small cell); lymphoma including Hodgkin's and Non-Hodgkin's lymphoma, melanoma, myeloma, neuroblastoma, oral cavity cancer (e.g. lip, tongue, mouth and/or pharynx), ovarian cancer, pancreatic cancer, prostate cancer, retinoblastoma, rhabdomyosarcoma, rectal cancer, renal cancer, cancer of the respiratory system, sarcoma, skin cancer, stomach cancer, testicular cancer, thyroid cancer, uterine cancer, cancer of the urinary system, and other carcinomas and sarcomas.

The NPs of the present invention are such that they are capable of delivering intracellularly an agent that directly or indirectly induces or prevents angiogenesis, and/or increases or decreases blood flow, in a subject or in a tissue. The term "directly" can mean that the agent *per se* has the required activity. The term "indirectly" can mean that the agent undergoes or mediates at least one further reaction to provide an agent effective for inducing or preventing angiogenesis, and/or increasing or decreasing blood flow, in a subject or in a tissue. The agent can be any of the therapeutic agents described herein, such as a protein, peptide, chemical compound, genetic material (i.e. an oligonucleotide) or any other active molecule. Preferably the therapeutic agent is an miRNA, such as the pro- or anti- angiogenic miRNAs described herein. The skilled person will appreciate how to construct a suitable delivery system comprising the therapeutic agents for any one of the NPs provided herein.

If the therapeutic agent is an oligonucleotide, it may be transferred to the cells of a subject or tissue to be treated by transfection, infection, microinjection, cell fusion, protoplast fusion or ballistic bombardment. The oligonucleotide may be administered to suitable cells, such as endothelial cells, *ex vivo* or *in vitro,* and the oligonucleotide-containing cells then transplanted into the mammalian host, as hereinbefore described.

The delivery systems may be used to provide the NPs (and thereby the therapeutic agent that is complexed to the NPs) without the need to use conventional pharmaceutical vehicles such as those required in tablets, capsules or liquids, as described herein.

It will be appreciated that the therapeutic agent attached to the NP may act as the active agent in medicaments and compositions of the invention. The NP conjugates and transfected cells may be used in a medicament which may be used in a monotherapy (i.e. use of the active agent) for treating, ameliorating or preventing a myocardial infarction, ischaemia or cancer. Alternatively, the NP conjugates and transfected cells may be used as an adjunct to, or in combination with, known therapies which may be used for treating, ameliorating or preventing a myocardial infarction, ischaemia or cancer. For example, in certain embodiments the treatment of cancer involves administering NP conjugates or transfected mammalian cells of the invention alone. In certain embodiments the treatment further includes administering to the subject an anti-cancer medicament or treatment e.g. chemotherapeutic agents or radiation. There may be synergistic therapeutic benefits for the co-use of a NP conjugate or transfected cell as described herein and a known medicament. For example, the possibility of developing a therapy that uses a lower concentration of a known medicament in order to avoid toxicity or unpleasant side effects associated with higher dosage rates may be achieved.

The NP conjugates and/or transfected cells may be combined in compositions having a number of different forms depending, in particular, on the manner in which the composition is to be used. Thus, for example, the composition may be in the form of a powder, tablet, capsule, liquid, ointment, cream, gel, hydrogel, aerosol, spray, micellar solution, transdermal patch, liposome suspension or any other suitable form that may be administered to a person, animal, tissue or cell in need of treatment. It will be appreciated that the vehicle of medicaments according to the invention should be one which is well-tolerated by the subject to whom it is given or tissue or cell to which it is applied.

Medicaments comprising NP conjugates or transfected cells according to the invention may be used in a number of ways. For instance, oral administration may be required, in which case the NP conjugates, for example, may be contained within a composition that may, for example, be ingested orally in the form of a tablet, capsule or liquid. Compositions comprising NP conjugates or transfected cells of the invention may be administered by inhalation (e.g. intranasally or orally). Compositions may also be formulated for topical use. For instance, creams or ointments may be applied to the skin.

NP conjugates and transfected cells according to the invention may also be incorporated within a slow- or delayed-release device. Such devices may, for example, be inserted on or under the skin, and the medicament may be released over weeks or even months. The device may be located at least adjacent the treatment site. Such devices may be particularly advantageous when long-term treatment with NP conjugates or transfected cells used according to the invention is required and which would normally require frequent administration (e.g. at least daily injection).

In a preferred embodiment, medicaments and compositions according to the invention may be administered to a subject by injection into the blood stream or directly into a site requiring treatment. For example, the medicament may be injected intramuscularly. Injections may be intravenous (bolus or infusion) or subcutaneous (bolus or infusion), or intradermal (bolus or infusion).

It will be appreciated that the amount of NP conjugates or transfected cells required is determined by their biological activity and bioavailability, which in turn depends on the mode of administration, the physiochemical properties of the conjugates and cells and whether they are being used as a monotherapy or in a combined therapy. The frequency of administration will also be influenced by the half-life of the active agents within the subject being treated. Optimal dosages to be administered may be determined by those skilled in the art, and will vary with the particular NP conjugates or cells in use, the strength of the pharmaceutical composition, the mode of administration, and the advancement of the ischaemic, cancerous or other disease. Additional factors depending on the particular subject being treated will result in a need to adjust dosages, including subject age, weight, gender, diet and time of administration.

Generally, a daily dose of between 0.001 µg/kg of body weight and 10 mg/kg of body weight of the NP conjugates or transfected cells according to the invention may be used for treating, ameliorating, or preventing a myocardial infarction, ischaemic disease, disorder or condition or cancer, depending upon which active agent is used. More preferably, the daily dose is between 0.01 µg/kg of body weight and 10 mg/kg of body weight, more preferably between 0.01 µg/kg of body weight and 1 mg/kg of body weight, even more preferably between 0.1 µg/kg and 100 µg/kg body weight, and most preferably between approximately 0.1 µg/kg and 10 µg/kg body weight.

The NP conjugates or transfected cells may be administered before, during or after onset of the disease, disorder or condition to be treated. Daily doses may be given as a single administration (e.g. a single daily injection). Alternatively, the NP conjugates or transfected cells may require administration twice or more times during a day. As an example, NP conjugates or transfected cells may be administered as two (or more, depending upon the severity of the disease) daily doses of between 0.07 µg and 700 mg (i.e. assuming a body weight of 70 kg). A patient receiving treatment may take a first dose upon waking and then a second dose in the evening (if on a two dose regime) or at 3- or 4-hourly intervals thereafter. Alternatively, a slow release device may be used to provide optimal doses of NP conjugates or transfected cells according to the invention to a patient without the need to administer repeated doses.

Known procedures, such as those conventionally employed by the pharmaceutical industry (e.g. *in vivo* experimentation, clinical trials, etc.), may be used to form specific formulations of the NP conjugates or transfected cells according to the invention and precise therapeutic regimes (such as daily doses of the agents and the frequency of administration). The inventors believe that they are the first to suggest a NP formulation, based on the use of miRNAs, for treating a myocardial infarction, ischaemic disease, disorder or condition or cancer.

Hence, in a further aspect of the invention, there is provided a myocardial infarction, ischaemic disease or cancer treatment composition comprising a therapeutically effective amount of a NP conjugate or transfected cell as described herein and, optionally, a pharmaceutically acceptable vehicle.

The term "myocardial infarction, ischaemic disease or cancer treatment composition" can mean a pharmaceutical formulation used in the therapeutic amelioration, prevention or treatment of a myocardial infarction, ischaemic disease or cancer.

The invention also provides in a further aspect, a process for making the composition according to the previous aspect, the process comprising combining a therapeutically effective amount of NP conjugates or transfected cells, with a pharmaceutically acceptable vehicle.

The NP conjugates and transfected cells used in the described therapeutic applications may be any of those described herein. Preferably, the NP conjugate comprises a core of PLGA and PFCE, coated with PS. Preferably the therapeutic agent is an miRNA. Preferably the transfected cells are endothelial cells, MNCs, cardiac progenitor cells or bone marrow cells.

A "subject", as used herein, may be a vertebrate, mammal or domestic animal. Hence, medicaments according to the invention may be used to treat any mammal, for example livestock (e.g. a horse), pets, or may be used in other veterinary applications. Most preferably, the subject is a human being.

A "therapeutically effective amount" of active agent (i.e. the NP conjugate or therapeutic agent comprised in the same) is any amount which, when administered to a subject, is the amount of drug that is needed to treat, prevent or ameliorate a myocardial infarction, ischaemic disease or cancer, or produce the desired effect.

For example, the therapeutically effective amount of NP conjugate or therapeutic agent may be from about 0.001 µg to about 1 mg, and preferably from about 0.01 µg to about 100 µg. It is preferred that the amount of NP conjuagte or therapeutic agent is an amount from about 0.1 µg to about 10 µg, and most preferably from about 0.5 µg to about 5 µg.

A "pharmaceutically acceptable vehicle" as referred to herein, is any known compound or combination of known compounds that are known to those skilled in the art to be useful in formulating pharmaceutical compositions.

In one embodiment, the pharmaceutically acceptable vehicle may be a solid, and the composition may be in the form of a powder or tablet. A solid pharmaceutically acceptable vehicle may include one or more substances which may also act as flavouring agents, lubricants, solubilisers, suspending agents, dyes, fillers, glidants, compression aids, inert binders, sweeteners, preservatives, dyes, coatings, or tablet-disintegrating agents. The vehicle may also be an encapsulating material. In powders, the vehicle is a finely divided solid that is in admixture with the finely divided active agents according to the invention. In tablets, the active agent (i.e. the NP conjugate or therapeutic agent) may be mixed with a vehicle having the necessary compression properties in suitable proportions and compacted in the shape and size desired. The powders and tablets preferably contain up to 99% of the active agents. Suitable solid vehicles include, for example calcium phosphate, magnesium stearate, talc, sugars, lactose, dextrin, starch, gelatin, cellulose, polyvinylpyrrolidine, low melting waxes and ion exchange resins. In another embodiment, the pharmaceutical vehicle may be a gel and the composition may be in the form of a cream or the like.

However, the pharmaceutical vehicle may be a liquid, and the pharmaceutical composition may be in the form of a solution. Liquid vehicles are used in preparing solutions, suspensions, emulsions, syrups, elixirs and pressurised compositions. The active agent according to the invention (NP conjugate or therapeutic agent) may be dissolved or suspended in a pharmaceutically acceptable liquid vehicle such as water, an organic solvent, a mixture of both or pharmaceutically acceptable oils or fats. The liquid vehicle can contain other suitable pharmaceutical additives such as solubilisers, emulsifiers, buffers, preservatives, sweeteners, flavouring agents, suspending agents, thickening agents, colours, viscosity regulators, stabilisers or osmo-regulators. Suitable examples of liquid vehicles for oral and parenteral administration include water (partially containing additives as above, e.g. cellulose derivatives, preferably sodium carboxymethyl cellulose solution), alcohols (including monohydric alcohols and polyhydric alcohols, e.g. glycols) and their derivatives, and oils (e.g. fractionated coconut oil and arachis oil). For parenteral administration, the vehicle can also be an oily ester such as ethyl oleate and isopropyl myristate. Sterile liquid vehicles are useful in sterile liquid form compositions for parenteral administration. The liquid vehicle for pressurised compositions can be a halogenated hydrocarbon or other pharmaceutically acceptable propellant.

Liquid pharmaceutical compositions, which are sterile solutions or suspensions, can be utilised by, for example, subcutaneous, intrathecal, epidural, intraperitoneal, intravenous and particularly intramuscular injection. The NP conjugates or transfected cells may be prepared as a sterile solid composition that may be dissolved or suspended at the time of administration using sterile water, saline, or other appropriate sterile injectable medium.

The NP conjugates, cells and other compositions of the invention may be administered orally in the form of a sterile solution or suspension containing other solutes or suspending agents (for example, enough saline or glucose to make the solution isotonic), bile salts, acacia, gelatin, sorbitan monoleate, polysorbate 80 (oleate esters of sorbitol and its anhydrides copolymerized with ethylene oxide) and the like. The agents used according to the invention can also be administered orally either in liquid or solid composition form. Compositions suitable for oral administration include solid forms, such as pills, capsules, granules, tablets, and powders, and liquid forms, such as solutions, syrups, elixirs, and suspensions. Forms useful for parenteral administration include sterile solutions, emulsions, and suspensions.
All of the features described herein (including any accompanying claims, abstract and drawings), and/or all of the steps of any method or process so disclosed, may be combined with any of the above aspects in any combination, except combinations where at least some of such features and/or steps are mutually exclusive.

In order to facilitate understanding of the appended examples, the following acronyms will be used:

| **Acronym** | |
|---|---|
| NPs | Nanoparticles |
| PLGA | Poly(lactic acid-co-glycolic acid) |
| PLGA-PFCE | Poly(lactic acid-co-glycolic acid)-Perfluoro-1,5-crown ether nanoparticle conjugate |

| **Acronym** | |
|---|---|
| *PLGA-PFCE | Fluorescent-labeled Poly(lactic acid-co-glycolic acid)-Perfluoro-1,5-crown ether nanoparticle conjugate |
| PS | Protamine sulphate |
| PLGA-PFCE-PS | Conjugate of Poly(lactic acid-co-glycolic acid)-Perfluoro-1,5-crown ether nanoparticles coated with protamine sulphate |
| *PLGA-PFCE-PS | Conjugate of fluorescent-labeled Poly(lactic acid-co-glycolic acid)-Perfluoro-1,5-crown ether nanoparticles coated with protamine sulphate |
| SPIO | Superparamagnetic iron oxide |
| *SPIO | Fluorescent-labeled superparamagnetic iron oxide nanoparticles |
| SPIO-PS | Superparamagnetic iron oxide nanoparticles coated with protamine sulphate |
| *SPIO-PS | Fluorescent-labeled superparamagnetic iron oxide nanoparticles coated with protamine sulphate |
| PLGA-PFCE- miRNA | Conjugate of Poly(lactic acid-co-glycolic acid)-Perfluoro-1,5-crown ether nanoparticles complexed with miRNA |
| PLGA-PFCE-PS-miRNA | Conjugate of Poly(lactic acid-co-glycolic acid)-Perfluoro-1,5-crown ether nanoparticles coated with protamine sulphate and complexed with miRNA |
| *PLGA-PFCE-PS-miRNA | Conjugate of fluorescent-labeled poly(lactic acid-co-glycolic acid)-Perfluoro-1,5-crown ether nanoparticles coated with protamine sulphate and complexed with miRNA |
| FITC | Fluorescein isothiocyanate |
| FITC-PLGA-PFCE | Conjugate of Poly(lactic acid-co-glycolic acid)-perfluoro-1,5-crown ether nanoparticles labeled with fluorescein isothiocyanate |

### Examples

The invention will now be described by way of illustration only in the following examples:

### Example 1: Isolation of MNCs from UCB

All human UCB samples were collected from donors, who signed an informed consent form, in compliance with Portuguese legislation. The collection was approved by the ethical committee of Hospital Infante D. Pedro. The samples were stored in sterile bags containing 35 ml of citrate-phosphate-dextrose anticoagulant solution. MNCs were obtained from UCB samples after Ficoll (Histopaque-1077 Hybri Max; Sigma-Aldrich, St. Louis, USA) density gradient separation. MNCs were immediately used for experiments without further treatment.

### Example 2: Preparation of fluorescent-labeled NPs

### 2.1 Preparation of fluorescent-labeled NPs containing fluorine-imaging agent [*PLGA-PFCE]

PLGA NPs were prepared by a single emulsion protocol. PLGA (Resomers® 502 H; 50:50 lactic acid: glycolic acid) (Boehringer Ingelheim) was covalently conjugated to fluoresceinamine (Sigma-Aldrich) according to a protocol reported by Horisawa et al. (2002) Pharm Res 19: 132-139. The fluoresceinimine acts as a simple reporter to follow NP delivery into cells. The NPs were prepared by dissolving the fluorescent-labeled PLGA (100 mg) in a solution of dichloromethane: trifluoroethanol (1:8, 6.3 ml) containing PFCE (100 mg) (Fluorochem, UK). This solution was then added dropwise to a PVA solution (40 ml, 5% w/v in water) and stirred for 3 hours. The *NPs were then centrifuged and washed with distilled water before freeze-drying.

### 2.2 Preparation of fluorescent-labeled SPIO NPs

Fluorescent-labeled SPIO NPs were used as a control. SPIO NPs commercialised as Endorem [Chu W. et al. (1995) Magn Reson Imaging 13: 661-674] (size 80-160 nms) were kindly donated by Guebert. The SPIO NPs were labeled with fluoresceinimine primarily as reported in Lee et al. (2008) Adv Mater Deerfield 20: 2512-2516, with some modifications. Briefly, SPIO NPs (approximately 10 mg in 1 ml of 10 mM citrate buffer pH 5.5) were incubated with sodium periodate (10 mg) for 2 hours at room temperature. The solution was ultra-centrifuged at 41,000 rpm for 15 minutes. The supernatant was discarded and the pellet was washed twice with distilled water. The remaining pellet was added to a solution of fluoresceinamine (2 mg/ml) in cyanoborohydride pH 8 (50 mM) and reacted for 2 hours at room temperature. The NP suspension was then ultra-centrifuged at 41,000 rpm for 15 minutes and once again washed with distilled water, re-suspended and lyophilised.

### Example 3: Coating of NPs with protamine sulphate (PS)

In some cases, NPs were coated with PS as described below.

A titration of PS amounts for coating PLGA-PFCE NPs was performed by incubating PLGA-PFCE NPs (1 mg/ml in PBS) and PS (at variable concentration according to the following table) for 10 minutes under agitation, at room temperature. After the incubation period the PLGA-PFCE-PS NPs were centrifuged (4200 rpm, 15 min) and resuspended in distilled water. This cycle was repeated two times and, after taking the measurements indicated in Table 1, the PLGA-PFCE-PS NP suspension freeze-dried.

**Table 1:**

| **PS (µg/ml in PBS)** | **Zeta (mV)** | **Polydispersion Index (PDI)** | **Size (nm)** |
|---|---|---|---|
| 0 | -10.56 ± 2.91 | 0.083 | 139.53 ± 2.74 |
| 1 | -9.67 ± 3.62 | 0.692 | - |
| 10 | -10.02 ± 2.46 | 0.143 | - |
| 100 | -8.92 ± 1.83 | 0.262 | - |
| 1000 | +9.83 ± 1.67 | 0.174 | - |
| 2000 | +7.65 ± 2.59 | 0.215 | 148.95 ± 1.94 |

All zeta potential and size measurements were performed in 10 mM KCl at pH 5.5. Results are expressed as average ± standard deviation, for n=3. For the zeta potential measurements, all data were recorded with at least six runs with a relative residual value (measure of data fit quality) of 0.03.

### 3.1 Coating of fluorescent-labeled PLGA-PFCE NPs

*PLGA-PFCE NPs prepared in example 2.1 above (1 mg/ml) were incubated with PS (1 mg/ml) for 10 minutes under agitation, at room temperature. After the incubation period, the NPs were dialysed (MWCO of 50 kDa) against distilled water and freeze-dried.

### 3.2 Coating of fluorescent-labeled SPIO NPs

*SPIO NPs prepared in example 2.2 (1 mg/ml) above were incubated with PS (1 mg/ml) for 10 minutes under agitation, at room temperature. After the incubation period, the NPs were washed by centrifugation with distilled water in cycles of maximum centrifugation speed for 15 minutes, later re-suspended in distilled water, and freeze-dried.

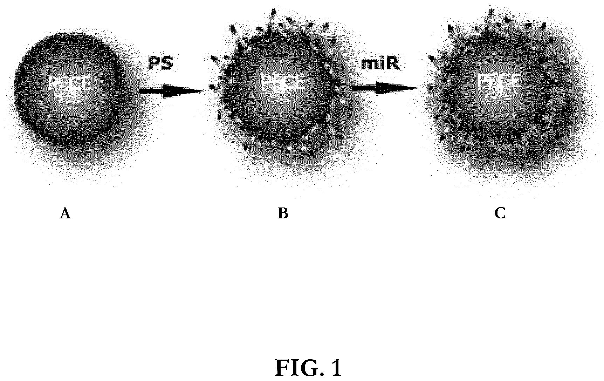

A schematic representation of the NP preparation and coating described in Examples 2 and 3 above is illustrated in Figure 1. In Step (A) of Figure 1, the NPs are formed by PLGA encapsulating PFCE. In Step (B) of Figure 1, the NPs are then coated with a polycationic agent, PS, which has a dual role: (i) to facilitate cell internalisation and (ii) to mediate the complexation of miRNAs. The complexation of the coated NPs to miRNAs (Step C) is discussed in Example 8 below.

### Example 4: Characterisation of NPs

### 4.1 Dynamic Light Scattering

Particle size was determined using light scattering via Zeta PALS Zeta Potential Analyzer and ZetaPlus Particle Sizing Software, v. 2.27 (Brookhaven Instruments Corporation). NPs suspended in 10 mM potassium chloride (KCl) buffer pH 5.5 (500 µg/ml) and sonicated for short times (<1 minute) were used. Typically, all sizing measurements were performed at 25 °C, and all data were recorded at 90°, with an equilibration time of 5 minutes and individual run times of 120 s (5 runs per measurement). The average diameters described in this work are number-weighted average diameters. The zeta potential of NPs was determined in a 10 mM KCl pH 5.5 solution, at 25 °C. All data were recorded with at least 6 runs with a relative residual value (measure of data fit quality) of 0.03.

The results of this analysis are shown in Table 2 below:

**Table 2:**

| **NP** | **Diameter (nm)** | **Polydispersion Index (PDI)** | **Zeta potential (mV)** | **Recovery yield**(%)** |
|---|---|---|---|---|
| *PLGA | 169.8 ± 7.1 | 0.680 | -9.3±2.8 | 80.5±8.7 |
| *PLGA-PFCE | 212.9 ± 14.3 | 0.244 | -9.7±0.7 | 75.3±8.6 |
| *PLGA-PFCE-PS | 218.0±9.3 | 0.381 | +7.0±1.7 | 83.9±3.1 |
| SPIO | 89.2±3.4 | 0.267 | -2.9±0.9 | n/a |
| *SPIO | 82.7±2.5 | 0.271 | +3.8±0.1 | n/a |

| | | | | |
|---|---|---|---|---|
| All NPs marked * were fluorescently labeled with fluoresceinimine as described in examples 2.1 and 2.2. ** The recovery yield of NPs after production was calculated according to the following equation: recovery yield = (NP weight x 100)/(initial PLGA weight + mass of PFCE used). | | | | |

This dynamic light scattering analysis showed that PLGA NPs without PFCE had an average diameter of 170 nm and a negative (∼ -9 mV) zeta potential. The encapsulation of PFCE in the NPs increased their average diameter from 170 to 213 nm.

For the chosen ratio of NP:PS (1:1, i.e. 1000 µg NPs and 1000 µg PS) the *PLGA-PFCE-PS NPs do not significantly aggregate and have a shift in zeta potential from -9.7 ± 0.7 to 7.0 ± 1.7 mV. Approximately 13 µg PS was adsorbed per mg NPs as assessed by a standard ninhydrin assay.

### 4.2 Transmission electron microscopy (TEM) analysis

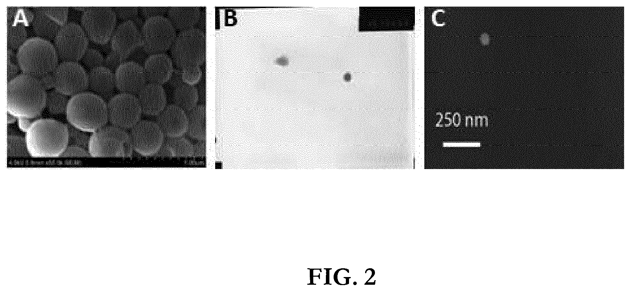

The morphology and size of the NPs was also evaluated by TEM (JEOL JEM-100 SX microscope at 80 kV) following negative staining with osmium tetroxide. A NP suspension (5 µl of PLGA-PFCE, 5 mg/ml in water) and osmium tetroxide solution (5 µl, 5% w/v) were placed on a 400 mesh copper grid with a carbon-coated Formvar membrane. The sample was then dried overnight before examination by TEM. The results of the TEM analysis of the PLGA-PFCE NPs are shown in Figure 2.

### 4.3 Mass loss of PLGA-PFCE

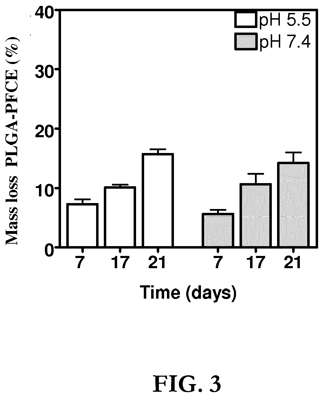

Mass loss of PLGA-PFCE NPs suspended in buffered solutions at pH 5.5 and 7.4 for several days, at 37 °C, was determined as followed. NPs (20 mg/ml) were re-suspended in PBS (pH 7.4) or KCl (pH 5.5) buffers and dialysed against the corresponding buffers at 37 °C. At specific time points (days 7, 17 and 21) the NP suspension was lyophilised and percentage of mass loss was calculated by subtracting the final mass from the initial mass. Results are shown as mean ± SD, *n*=3 in Figure 3.

### 4.4 Diameter of NPs over time

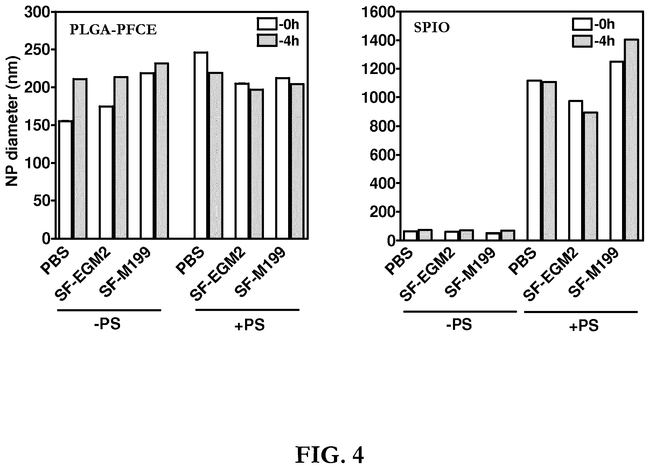

The diameter of PLGA-PFCE and SPIO NPs with and without the PS coating was measured. The NPs were suspended in PBS or culture media (serum free M199 and serum free EGM2 media) and the diameter was measured at time 0 and after 4 hours. The NPs were suspended at 1 mg/ml in the corresponding media and sonicated for 15 seconds before measurement (t = 0). Alternatively, the NPs were suspended at 1 mg/ml in the corresponding media and sonicated for 15 seconds, then incubated for 4 hours at 37 °C before measurement (t = 4 hours). The counts per second (Kcps) during the measurements were between 300-400 Kcps. The results are expressed as an average (*n*=3) in Figure 4.

### 4.5 Quantification of fluorine in NPs by ¹⁹F-NMR

¹⁹F NMR analyses were performed in a 600 MHz NMR Varian Instrument. PLGA-PFCE NPs (3 mg) were dissolved in dichloromethane (300 µl) and transferred to a 5 mm NMR tube. A sealed 3 mm tube containing trifluoroacetic acid (TFA) with ¹H-dichloromethane was inserted in the 5 mm and used as an internal reference. A total of 50 scans were run, with pulses at 90° after a relaxation time of 15 seconds.

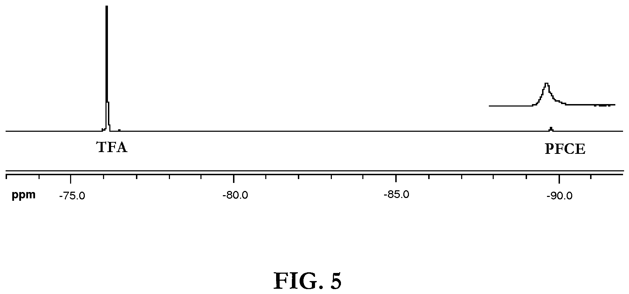

The ¹⁹F NMR spectrum of the PLGA-PFCE NPs dissolved in dichloromethane is shown in Figure 5. The concentration of fluorine was calculated from the ratio of the peak area at δ-89 ppm (fluorine in PFCE) and the one at -76 ppm corresponding to a known concentration of the internal reference (TFA). The results are shown in Table 3 below.

**Table 3:**

| **NP Formulation** | **PFCE (µg per mg PLGA)** | **Encapsulation efficiency (%, w/w)*** | **Fluorine concentration (µg of F per mg of NPs)** |
|---|---|---|---|
| PLGA-PFCE (ratio of 3.6 mg PFCE: 1 mg PLGA) | 176.5 | 4.9% | 98.0 |
| PLGA-PFCE (ratio of 0.8 mg PFCE: 1 mg PLGA) | 111.0 | 13.8% | 65.3 |

| | | | |
|---|---|---|---|
| *Encapsulation efficiency (% w/w) = (experimental amount of PFCE in PLGA-PFCE NPs × 100) / initial amount of PFCE. | | | |

It can be seen from Table 3 that the encapsulation efficiency assessed by ¹⁹F NMR analysis was between 4.9% and 13.8%. The PLGA-PFCE formulation has between 111.0 and 176.5 µg per mg of PLGA depending on the initial PFCE concentration used for the preparation of the PLGA-PFCE NPs.

### 4.6 Quantification of fluorine in NP-labeled cells by ¹⁹F-NMR

Cells (2 × 10⁶ cells/condition) were labeled with the PLGA-PFCE NPs (between 500 µg/ml and 8 mg/ml) for 4 hours in serum free EGM-2 (HUVECs) or serum free M199 (MNCs). After incubation, HUVECs were washed 3 times with PBS and trypsinised (0.2% trypsin), while MNCs were washed 3 times with PBS and then passed through a MACS column (Miltenyi Biotec) to further separate the cells from the non-internalised NPs. At the end, all cells were counted with trypan blue, resuspended in PBS, frozen at 80 °C and freeze-dried. The lyophilised cells were then dissolved in ¹H-dichloromethane (300 µl), transferred to a 5 mm NMR tube and characterised by ¹⁹F NMR as described in Section 4.5 above.

### Example 5: Characterisation of intracellular delivery

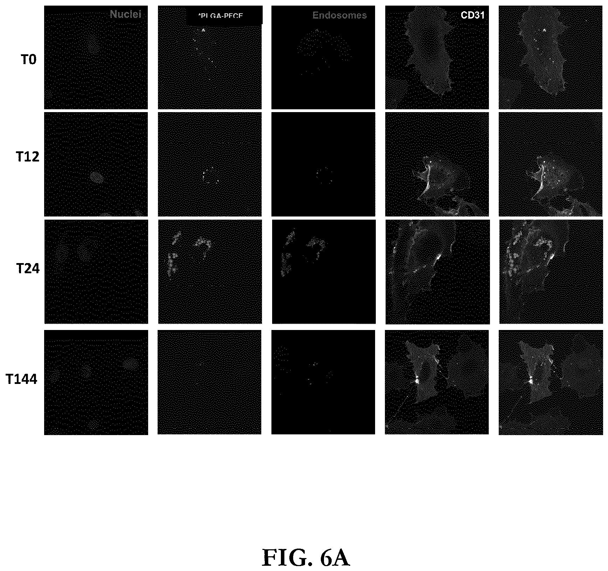

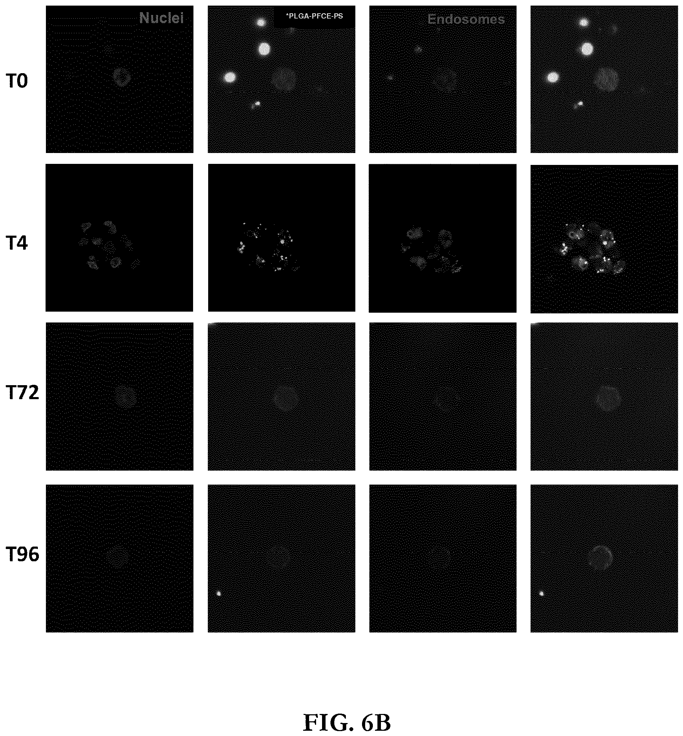

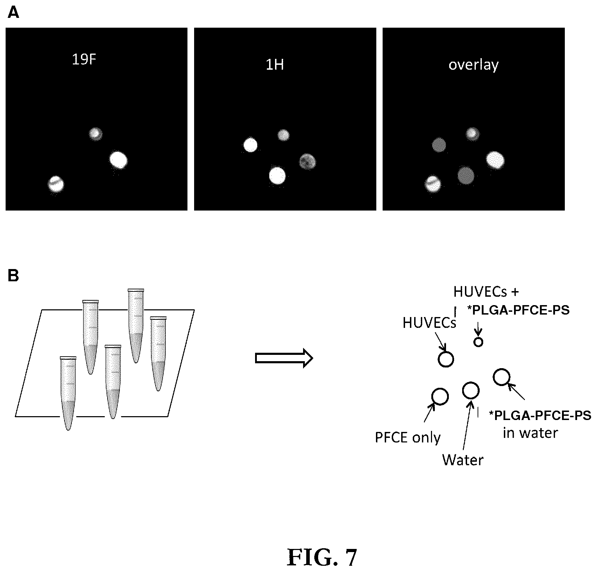

To characterise intracellular delivery of the fluorescent-labeled PLGA-PFCE NPs into the cytoplasm of HUVECs or cord blood MNCs, confocal laser scanning microscopy and MRI analyses were performed. *SPIO NPs were used as a control. Results are shown in Figures 6 and 7.

### 5.1 Confocal imaging

Cells were seeded onto circular 20 mm glass coverslips coated with 0.2% gelatin inside a 24 well plate and left to adhere. The cells were then incubated with 500 µg/ml of *PLGA-PFCE NPs or *SPIO NPs for 4 hours, after which the cells were washed with PBS, centrifuged and cultured in serum free EGM-2 (SF-EGM-2; HUVEC loading medium) or serum free M199 (SF-M199, MNC loading medium). Culture incubations were performed for 0, 12, 24, 72, 96 or 144 h (as indicated in Figure 6).

Once the incubations were terminated the coverslips were washed gently, in some conditions live staining for endolysosomal vesicles (using LysoTracker red DND-99 at 50 nM, 20 minute incubation) or mitochondrial activity (using MitoTracker Red CM-Ros, 50 nM, 15 minute incubation) was done and rewashed prior fixation. Then, cells were fixed with 4% (w/v, in water) paraformaldehyde (EMS, Hatfield, USA) for 10 minutes at room temperature and then washed with PBS. For certain conditions, the slides were mounted straight away with mounting medium-4',6-diamidino-2-phenylindole (DAPI; Sigma-Aldrich) on a glass slide. However, in some conditions, cell membrane staining was performed. In this case, mouse antibodies against human CD31 (Dako, Glostrup, Denmark) or CD45 (BD Biosciences, Spain) were used to stain the membrane of HUVECs or MNCs. Cells were washed 3 times after fixation (MNCs were washed by centrifugation), the cells were then blocked with 2% bovine serum albumin (BSA) (Sigma-Aldrich) in PBS for 30 minutes at room temperature. The CD31/CD45 (both at 1:50 dilution) primary antibody was added in PBS for 1 hour at room temperature followed by 3 washes in PBS. The binding of primary antibodies was detected with anti-mouse DyLight 649 conjugate (at a dilution of 1:200) (Jackson ImmunoResearch, USA). The nucleus of cells was stained with mounting medium-DAPI. After the indirect labelling, the cells were examined with a Zeiss Laser Scanning Microscope Meta 510.

Transfection efficiency was evaluated by incubating cells grown on glass coverslips (4 h at 37 °C) with fluorescent miR-Dy547 (red) carried by fluorescent NPs (NP-fluorescein, green) or using SiPORT transfection agent. Low magnification (20x objective, Zeiss Laser Scanning Microscope Meta 510) photographs were taken observing the need for total signal capture (maximally opening the pinhole) and background normalisation. Quantification of the percentage of transfected cells was done by counting the number of miR-DY547 positive cells by comparison with the levels of fluorescence in untransfected cells using ImageJ. Representative microscopic fields were used for quantification spanning a total area of 4.3 mm2.

The total loading per cell was calculated with ImageJ as the "integrated density" that is given by the product between the mean fluorescence intensity and the area of the cell; 200 cells were analysed in each condition giving a total area of 0.250 mm².

Figure 6A shows confocal images illustrating the internalisation of NPs by HUVECs. DAPI stains the cell nuclei blue, LysoTracker stains the endosomes red and CD31 stains the cell membrane grey. The fluorescent labelling of the NPs is green. As can be seen from Figure 6A, the PLGA-PFCE formulation was taken up by HUVECs by an endocytic process, mediated in part by actin fibres, and accumulated substantially at the endosomes. This endosomal sequestration persisted for up to 144 hours following incubation (see T144 panels in Fig. 6A), indicating relatively little self-mediated escape or disruption of endosomal membranes by the NPs themselves. Similar results have been obtained for MNCs, as shown in Figure 6B.

### 5.2 MRI imaging

The MRI images are shown in Figure 7. Fig. 7(B) shows an illustration of PLGA-PFCE NPs (8 mg), PFCE (750µl), PLGA-PFCE-PS-labeled HUVECs (10 x10⁶ cells) and unlabeled HUVECs (10 x 10⁶ cells) and water contained in separate eppendorfs. All eppendorfs were positioned onto a support inside a ¹⁹F 7.0 T MRI volume coil. The images acquired (shown in Fig. 7(A)) were coronal slices in relation to the magnet orientation. The acquisition time was 10 minutes (5 averages).

### Example 6: Cell viability and long-term labelling

### 6.1 Quantification of labelling efficiency

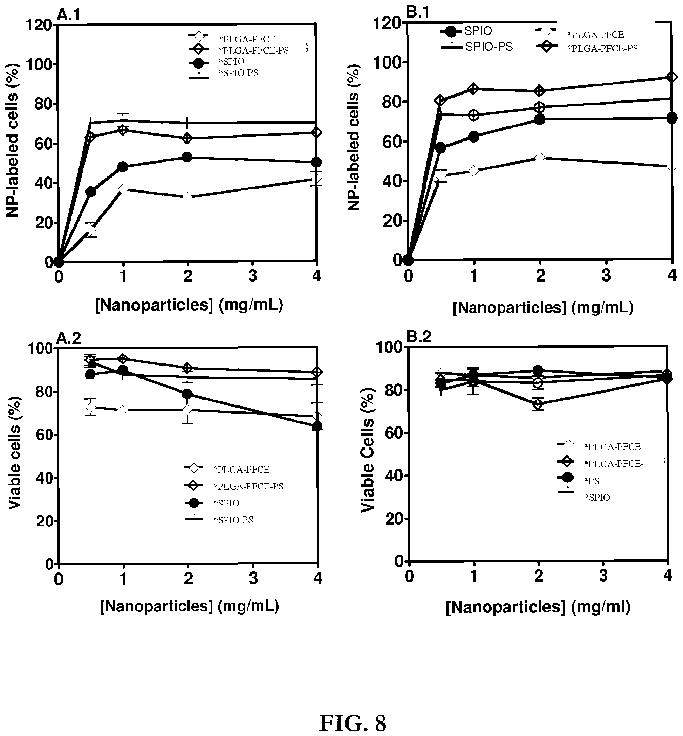

To quantify the labelling efficiency of the cells under the different conditions tested Fluorescence Activated Cell Sorting (FACS) was performed, and the results are shown in Figure 8 ((A1) & (A2) HUVECS; (Bi) & (B2) MNCs).

MNCs (1×10⁶ cells) or HUVECs (0.2×10⁶ cells) were incubated for 4 hours in serum free M199 or serum free EGM-2, respectively, containing variable concentration of NPs (between 0.5 and 4 mg/ml) in a 24 well plate. After 4 hours the HUVECs were washed with PBS on the plate 3 times to remove loosely bound particles. The cells were then re-suspended in serum free EGM-2 with or without mitotracker red CMX-ROS at 50 nM for 15 minutes at 37°C in a CO₂ incubator. The cells were later trypsinised with 0.2% (w/v) trypsin solution, centrifuged at 1000 rpm for 5 minutes, and fixed with 4% paraformaldehyde for 10 minutes at room temperature. After fixation they were rewashed and then re-suspended in 500 µl of PBS, ready for FACS analysis. MNCs were transferred to a 2 ml eppendorf and centrifuged for 15 minutes at 20°C, 1300 rcfs, then the MNCs were further washed by using anti-CD45 human microbeads via Miltenyi Biotec Mini MACS system and protocol (Miltenyi Biotec, UK) so as to ensure that only MNCs were present in the final solution and no free NPs, the cells were then incubated with mitotracker (50 nM) for 15 minutes at 37 °C, washed once with PBS and fixed with 4% paraformaldehyde for 10 minutes at room temperature. After fixation, the cells were washed and resuspended in 500 µl of PBS before FACS analysis. A total of 80,000 events were recorded per measurement.

As can be seen from Figure 8, when 500 µg/ml of PLGA-PFCE-PS was incubated with cells, 65% of HUVECs and 80% of MNCs were labeled after 4 hours. According to ¹⁹F-NMR (Figure 5) this labelling corresponds to the internalisation of 0.27 and 0.15 ng of PFCE per cell. This amount is enough for cells to be detected in less than 10 minutes using a 19F 7.0 T MRI volume coil. A greater amount of PS-coated PLGA-PFCE was taken up by the cells (approximately 3 times more) than uncoated ones. Interestingly, increasing the initial dose of NPs incubated with the cells did not increase the fraction of the cell population associated with fluorescence, indicative that a small percentage of cells (< 20%) were unable to taken up the NPs. Similar internalisation profiles were obtained for SPIO NPs.

### 6.2 Measurement of cytotoxicity of PLGA-PFCE formulation

To evaluate the cytotoxicity of *PLGA-PFCE formulation, HUVECs (in serum-free EGM2) and MNCs (M199) were exposed to variable concentrations of *PLGA-PFCE NPs or *SPIO NPs (as a control) for 4 hours.

At the end of the incubation, cells were washed and then cell viability was monitored by FACS using a Mitotracker CMX-ROS assay. This assay measures cell mitochondrial activity, which is directly proportional to cell viability, i.e cells positive for Mitrotracker CMX-ROS have mitochondrial activity and thus were considered as live cells. Results are shown in Figure 8 for HUVECs (see panel (A)) and MNCs (see panel (B)). Cells labeled with non-fluorescent NPs were used to define the gates during FACS acquisition. In all plots, results are average ± SD, *n*=3.

The *PLGA-PFCE NPs showed no substantial toxicity against endothelial cells and human cord blood-derived hematopoetic stem cells (Figure 8 and unpublished observations).

### 6.3 Effect of protamine sulphate concentration on cell viability

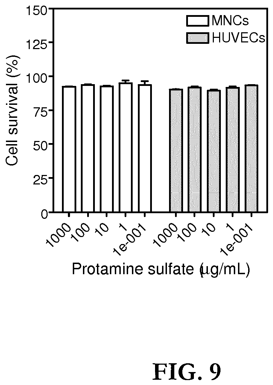

It was determined that HUVEC and MNC viability is not affected by PS at concentrations up to 1000 µg/ml (Figure 9).

### Example 7: Mechanism of NP uptake

### 7.1 Characterisation of endocytotic mechanism involved in the uptake of the NPs and long-term labelling of cells

To further elucidate the mechanism(s) of internalisation, the uptake of fluorescently labeled PLGA-PFCE by HUVECs and MNCs in the presence or absence of a variety of inhibitors for known endocytotic mechanisms was characterised. Both cell types (HUVECs: 2 × 10⁵ cells per condition; MNCs: 1× 10⁶ cells per condition) were labeled with *PLGA-PFCE-PS (500 µg/ml) in the presence or absence of chemical inhibitors, at 37 °C or 4 °C, before their characterisation by FACS. When inhibitors were used, the cells were pre-conditioned with the inhibitors for 1 hour prior to the addition of NPs for a further 4 hours. In the case of HUVECs, the cells were washed with PBS, trypsinised and fixed with 4% (w/v) paraformaldehyde for 10 minutes at room temperature. After fixation, the cells were washed and resuspended in 500 µl of PBS before FACS analysis. In case of MNCs, the cells were washed with PBS, separated from non-internalised NPs by MACS (using anti-human CD45 microbeads), fixed and finally characterised by FACS.

The inhibitors included cytochalasin D (Cyto D, 3 M; actin inhibitor), filipin (Fili, 3 g/ml; caveola inhibitor), and nocodazole (Noco, 10 µM; microtubule inhibitor).

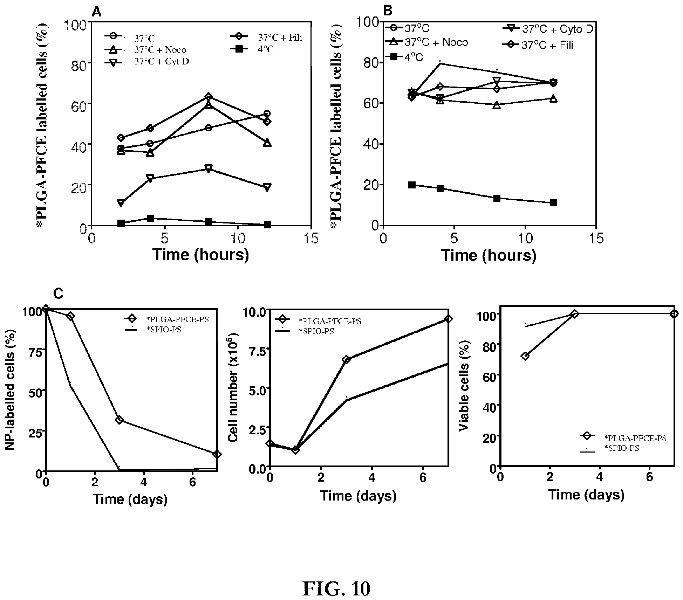

The results of the FACS analysis are shown in Figure 10(A) and 10(B). FACS results show strong inhibition of NP uptake by HUVECs and MNCs cultured at 4 °C, demonstrating the involvement of energy-dependent endocytotic mechanisms in the uptake of the NPs. Inhibition of NP uptake by HUVECs was also observed when cells were treated with Cyt D, although significantly less than that observed for cells cultured at 4 °C. Therefore, these results indicate the involvement of actin microfilaments in the active uptake of NPs by HUVECs. In contrast, no inhibition of NP uptake was observed when MNCs were treated with Cyt D indicating differences in the mechanism of NP internalisation.

### 7.2 Cell NP labelling over time

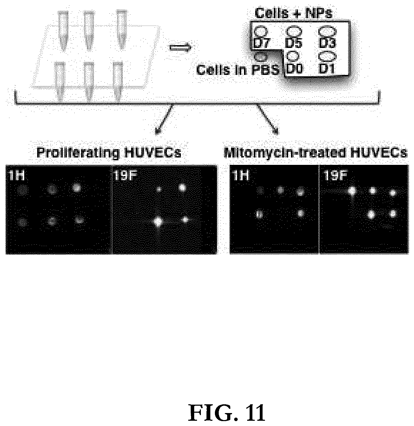

Long-term observation of PLGA-PFCE-labeled stem cells might be limited because of dilution of NPs with cell division. Therefore, cell NP labelling was quantified over time by FACS (fluorescence quantification), as shown in Figure 10(C), and MRI, as shown in Figure 11.

For FACS analysis, HUVECs were labeled with PLGA-PFCE NPs for 4 hours, being the labeled cell population isolated by sorting and cultured for 7 days in EGM-2 medium.

For MRI, cells were transfected with PLGA-PFCE-PS (10 x 10⁶ cells with 8 mg NPs) and MRI images taken at time o (Do), after 1 day (D1), 3 days (D3), 5 days (D5) and 7 days (D7). MRI images were taken of 10 x 10⁶ cells in PBS as a control. Images were taken of the cells in separate eppendorfs. Both untreated and mitomycin-treated cells were used. All eppendorfs were positioned onto a support inside a 19F 7.4T MRI volume coil. The images acquired were coronal slices in relation to the magnet orientation. The acquisition time was 10 minutes (5 averages).

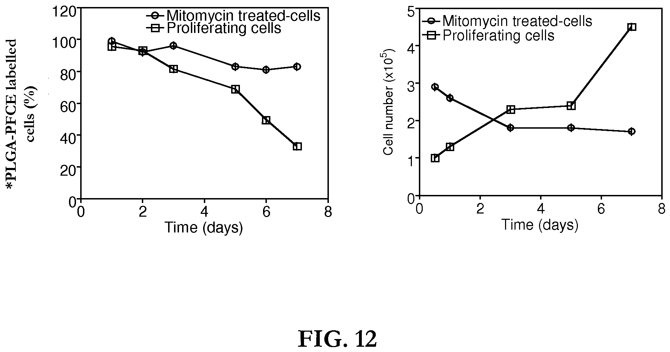

During the 7 day culture period for FACS analysis, cells proliferate and have viabilities above 90% at days 3 and 7 (Figure 10(C)). Curiously, SPIO-labeled cells proliferate less than PLGA-PFCE-labeled cells. Importantly, the percentage of PLGA-PFCE- and SPIO-labeled cells decreased over time. Only 30% of the cells were positive for the presence of PLGA-PFCE after 4.7 cell doublings while almost none of the cells were positive for the presence of SPIO during the same number of doublings (Figure 10). The decrease in PLGA-PFCE-labeled cells is due to cell division since inhibiting the cell cycle by mitomycin, the intracellular level of NPs remains fairly constant over 7 days (Figures 11 and 12).

### Example 8: Complexation of NPs with miRNAs and cell transfection

### 8.1 Pro-survival miRNA delivery

In order to determine whether the PLGA-PFCE formulation could be used to deliver pro-survival miRNAs within cells, the following procedure was followed. Firstly, three different miRNAs were selected: miR-132 (Ambion), miR-424 (Ambion) and amiR-92a (antagomir-92a (Exiqon)), for complexation with FITC-labeled PLGA-PFCE as follows. FITC-PLGA-PFCE NPs were weighed, sterilised under UV light for 30 minutes, and resuspended in EGM-2 serum free medium to yield a final concentration of 500 µg/ml. The suspension of NPs was then dispersed by ultrasound (2 × 10 s, BRANSON 2510), and miRNAs added (100nM and 200 nM) and allowed to complex to the NPs for 1 hour at 37 °C, with intermittent agitation. Cells were washed 2 times with pre-warmed PBS and the NP-miRNA conjugate was added to the cells for incubation at 37 °C for 4 hours. Cells were transfected keeping a ratio of 1.25 mg of NP-miRNA per million HUVEC cells.

To evaluate the potential of our approach, we performed the same experiment using a commercial transfection agent- siPORT NeoFX. In a SiPORT (Ambion) transfection procedure, SiPORT transfection agent and miRs were diluted in EGM-2 serum free medium and incubated at room temperature separately. After 10 minutes, both solutions were combined and incubated for another 10 minutes to allow the complexation of both components. The suspension was then dispensed on top of PBS-pre-washed cells and incubated for 4 hours at 37 °C, as in the PLGA procedure.

### 8.2 Confocal imaging

For transfection visualisation under the fluorescence microscope FITC-PLGA-PFCE NPs and miRIDIAN microRNA mimic transfection control with Dy547 (miR-Dy547) (Dharmacon) (100 nm) were used. Dy547-labeled miRNA is a mimic miRNA without any human targets (no fluorescent miR132 and miR424 could be obtained commercially).

PLGA-PFCE NPs (FITC-labeled or unlabeled) or siPORT complexed with validated fluorescent oligonucleotides (200 nM miR-Dy547 or fluorescent-labeled amiR-92a) were incubated with HUVECS (2 x 10⁵ cells) for 4 h at 37 °C. The transfected cells were then washed with PBS to remove NPs that were not internalised, fixed and immunolabeled as described previously (Horisawa et al. (2002) Pharm Res 19: 132-139). Briefly, the cell membranes were permeabilised with tritonX-100/ PBS (0.5%, v/v), non-specific protein interactions were blocked with BSA (2%, w/v) plus fetal calf serum (FCS) in PBS (2%, v/v) and the cells were incubated with primary antibodies according to manufacturer's instructions (overnight staining at 4°C with anti-human Ago2 (1B1-E2H5 clone active motif) 1:500 and anti-human GW182 (H70, santa cruz biotechnology sc-66915) 1:100. Other antibodies were incubated for 1 h at room temperature; namely, the early endosomal marker EEA1 (Cell Signaling C45B10, 1:200 dilution) and the late endosome/lysosome marker protein RAB7 (Cell Signaling D95F2, 1:100 dilution). Secondary antibodies were anti-mouse Cy3 (Sigma C2181), anti-rabbit Cy3 and anti-mouse DyLight 649 (Jackson ImmunoResearch). The nucleus of the cells was stained with DAPI.

After the indirect labelling, high magnification confocal images (60x objective) were taken this time using the optimal pinhole for better discrimination between foci and assuring no over-exposure or bleed-through between channels. Usually images were composed of 4 channels (blue, green, red and far-red) where different interactions were analysed. NPs were green or non-fluorescent depending on how many co-interactions were analysed, e.g. when assessing the association between miR-Dy547/Ag02/GW182 or amiR-92a/Ag02/GW182 non-fluorescent NPs were used. Images were exported to imajeJ and analysis was performed with stacks containing the different channels.

The interaction of the NPs and/or miRNAs with the different proteins was assessed considering positive associations to be within 500 nm distance between centres of foci using ImageJ. All the discrete foci (fluorescence levels higher than background) present inside the cell were analysed by overlaying a ruler of 500 nm length in all directions from the centre of each foci and scoring the number of foci in the other channels that are contained inside this area; at least 100 cells were analysed in each condition. Due to the highly variable number of foci present inside the cell (e.g. Ago2 low, EEA1 high, miRNAs high) we found automated co-localisation tools available in ImageJ not to be adequate for this type of analysis.

To evaluate the potential of our approach, we performed the same experiment using siPORT NeoFX. Representative microscopic fields were used for quantification spanning a total area of 4.3 mm².

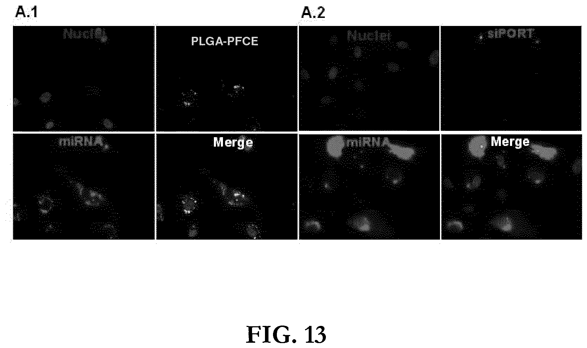

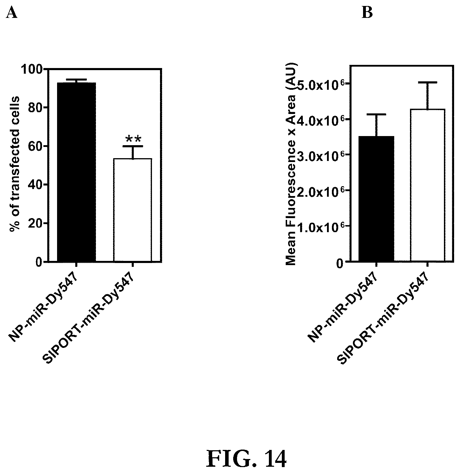

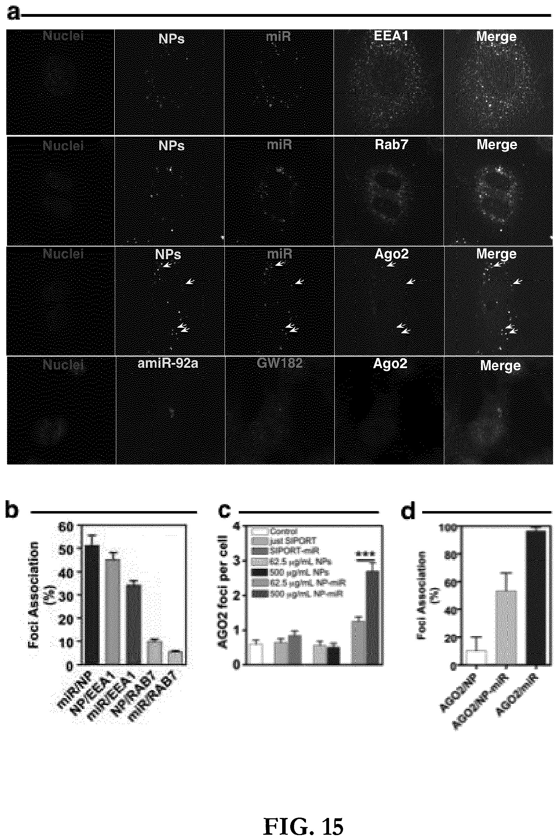

As can be seen from Figure 13, panel A1, the complex miR-Dy547: FITC-PLGA-PFCE was highly taken up by cells. Over 90% of the cells were transfected with miRNA (Figure 14(A)). FITC-PLGA-PFCE NPs taken up by HUVECs were in general localised in restricted areas of the cells (Figure 15(A)). Most of the miRNA co-localised with the particles, while a small amount of miRNA was distributed across the cell (Figure 13, panel A1).

Representative images of the intracellular distribution of FITC-PLGA-PFCE NPs and miR-Dy547 with respect to the early endosome (EEA1) and late endosome/lysosome (Rab7) vesicles are shown in Figure 15(A) (top panels). Representative images showing the association between FITC-PLGA-PFCE NPs and miR-Dy547 foci with the argonaute2 (Ago2) protein are shown in Figure 15(A) (bottom panels). The amiR-92a associates with both GW182 and Ago2 proteins.

Figure 15(B) illustrates the intracellular localisation of FITC-PLGA-PFCE NPs and miR-Dy547 foci with respect to the endomembrane system. The % association (y-axis) is a relative measure of the number of foci inside the cell (% of total) that are interacting with the species identified in the denominator (x-axis). At 24 h post-transfection, 45% of the FITC-PLGA-PFCE NPs remained in the early endosomes (EEA1⁺ vesicles) and were not substantially trafficked to late endosomes/lysosomes (Rab7⁺ vesicles, 10%). Approximately 50% of the miRNA co-localised with the particles, thus showing that half of the miRNA had already been released by the FITC-PLGA-PFCE NPs (Figure 15(B)). Approximately 35% of the miR-Dy547 foci were associated with early endosome vesicles (EEA1⁺ vesicles) while only 5% were positive for (i.e. trafficked to) late endosome and lysosome vesicles (Rab7⁺ vesicles). Overall, there was a low degree of association of both NP and miRNA with late endosome and lysosome vesicles.

Figure 15(C) provides a quantification of the number of Ago2 foci present inside the cell under different transfection conditions. The data show that the number of Ago2 foci present in the cell significantly increased with increased concentrations of NPs carrying miR-Dy547.

Figure 15(D) provides an analysis of the Ago2 association profile in PLGA-PFCE-miR-Dy₅₄₇-transfected cells. The figure provides a quantification of the degree of association of Ago2 foci and NP-only foci (Ag02/NP), NP foci and miRNA foci (Ag02/NP-miR), and foci containing just miRNA (Ag02/miR).

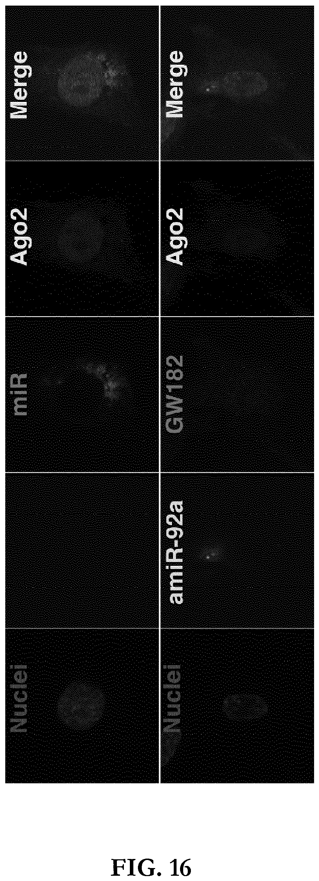

In contrast to the localised uptake of FITC-PLGA-PFCE NPs, and as can be seen from Figure 13, panel A2 and Figure 16, the miR-Dy547 released by siPORT NeoFX was distributed across the cell cytoplasm and not in confined intracellular areas. Importantly, FITC-PLGA-PFCE NPs were able to present miRNAs to the RISC protein, Ago2, more efficiently than siPORT. NP delivery induced more Ago2 foci than siPORT delivery. The same was observed when fluorescent amiR-92a was transfected. AmiR-92a foci showed association with both Ago2 and GW182 proteins, indicating that this anti-miR oligonucleotide may be capable of microRNA-RISC strand invasion. Representative photographs of siPORT delivery of miR-Dy547 and amiR-92a are shown in Figure 16 (top and bottom panels, respectively). It can be seen that both oligonucleotides failed to interact with Ago2 (amiR-92a data not shown). In addition, amiR-92a showed no association with GW182 using the siPORT delivery system.

Furthermore, only 50% of the cells were transfected with siPORT-Dy547 miRNA (Figure 14(A)). Figure 14(B) shows the total loading (integrated density = mean fluorescence x area) of miR-Dy547 in transfected cells (area of 0.250 mm²; approximately 200 cells were analysed); there was no significant difference in total loading between the two delivery vehicles (FITC-PLGA-PFCE NPs and siPORT).

Overall, our results show that HUVECs internalise the miRNAs and their intracellular distribution is dependent on the NP formulation.

### 8.3 Pro-survival activity

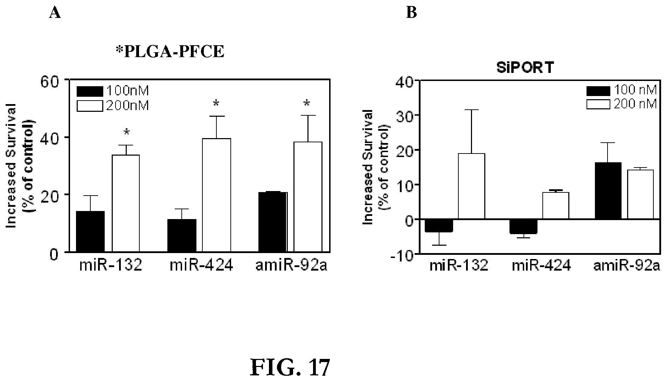

To demonstrate the pro-survival activity of different miRNAs formulations, HUVECs were incubated for 4 hours with miRNA-complexed NPs, washed, and finally cultured in ischaemic conditions (pO₂ of 0.1%; media without glucose) for 48 hours. Survival was assessed by an ATP-based assay. Both miR-132, miR-424 and amiR-92a PLGA-PFCE NP formulations contribute significantly (*P*<0.05) to the survival of the cells, and this effect is dependent on the concentration of miRNA (Figure 17(A)). No significant differences exist between the different miRNAs tested (*P*>0.05). Interestingly, miRNAs delivered by siPORT transfection agent mediates cell survival only for higher concentrations than the ones observed for PLGA-PFCE (Figure 17(B)). In both graphs results are shown as average ± SEM (n=3); results were considered significant when P ≤ 0.05 (*) and P ≤ 0.01 (**).

### 8.4 Survival and angiogenesis assays

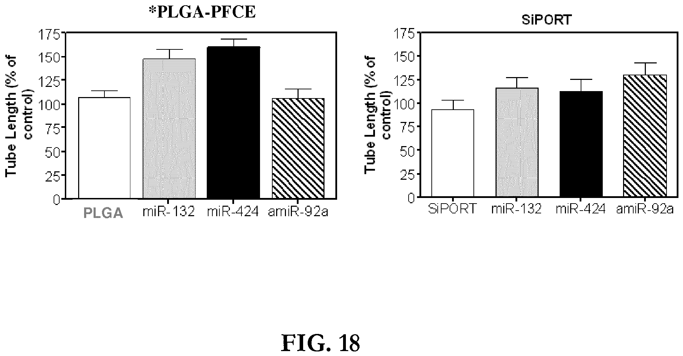

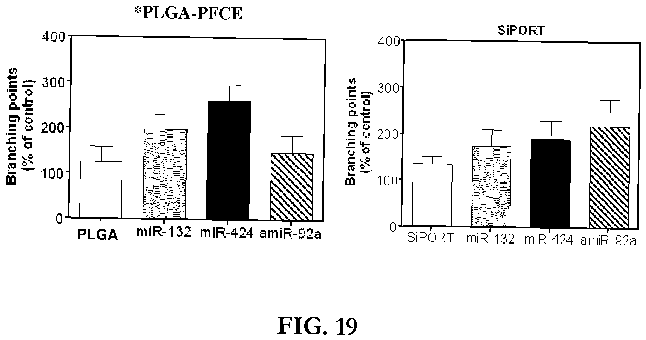

To demonstrate the pro-angiogenic effects of the miRNA-containing NP formulations, HUVECs were incubated for 4 hours with miRNA-complexed NPs, washed, and finally cultured on top of Matrigel to assess their capacity to form vascular networks. After transfection cells were washed 3 times with warm PBS and were left in complete EGM2 medium overnight to recover from transfection procedures. Next morning the cells were washed with PBS and survival challenge was done by incubating cells under oxygen (0.1%) and serum deprivation conditions for another 48 hours. Survival was assessed using the ATP-based assay Cell Titer Glow (Promega). *In vitro* angiogenesis assay was conducted using micro-angiogenesis slides (IBIDI) and Matrigel (BD) according to manufacturer instructions. After 24 hours tube length and number of branching points was measured (in a blind way) and compared between conditions. The transfection of the cells with miR-132- and miR-424-containing PLGA-PFCE NPs yielded vascular networks with higher tube length and branching points than cells transfected with amiR-92a (Figure 18, Figure 19). In contrast, the transfection of the cells with siPORT containing amiR-92a yielded vascular networks with higher tube length and branching point than cells transfected with miR-132 and miR-424. In all graphs results are shown as average ± SEM (n=3); results were considered significant when P ≤ 0.05 (*) and P ≤ 0.01 (**).

It is believed that the accumulation of miRNA in the endolysosomal compartment, as demonstrated in Example 8.2, may explain the higher pro-survival and pro-angiogenic activities achieved for the delivery of miR-132, miR-424 and amiR-92a by PLGA-PFCE NPs than siPORT carrier, which accumulates in the cytoplasm.

### Example 9: Intracellular delivery of miRNA

This study focused on the mechanism underlying the presentation of miRNA from the NPs in the endolysosomal compartment. From all the miRNAs tested miR132 was selected for subsequent analysis.

miR132 is expressed by endothelial cells after 3-6 h of exposure to vascular endothelial growth factor or basic fibroblast growth factor (Anand et al. (2010) Nat Med 16: 909-914). In addition, the constitutive expression of miR-132 in HUVECs considerably increases cell proliferation and tube formation. The therapeutic activity of miR132 has recently been demonstrated after the transplantation of saphenous vein-derived pericyte progenitor cells in a mouse myocardial infarction animal model, where it was shown that miR132 released from the transplanted cells stimulated endothelial tube formation by a Ras-dependent induction mechanism which in turn activated the PI3K/Akt pathway (Katare et al. (2011) Circ Res 109: 894-906).

One of the identified targets of miR-132 is Rasa1, which encodes RAS p21 protein activator (GTPase activating protein) 1 (p120RasGAP). HUVECs transfected with miR132 have a decreased endogenous p120RasGAP expression, which increases Ras activity and mitogen-activated protein kinase extracellular related protein kinase-i (MEK-1). To demonstrate that Rasa1 is indeed downregulated in HUVECs transfected with PLGA-PFCE-PS-miR132, a quantitative real time polymerase chain reaction (qRT-PCR) analysis was performed, as set out below.

This analysis was complemented by investigating the expression of another predicted target of miR-132, EDNRA, which encodes the receptor for endothelin 1, endothelin receptor type A.

HUVECs were incubated for 4 h with PLGA-PFCE-PS-miR132 or PLGA-PFCE-PS-scrambled miRNA or only PLGA-PFCE-PS, washed, and the gene expression profile of the specific targets of miR-132 was evaluated after 24 h. In this regard, total RNA from experimental groups was isolated using a protocol with TRIzol (Invitrogen) and RNeasy Minikit (Qiagen, Valencia). cDNA was prepared from 1 mg total RNA using Taqman Reverse transcription reagents (Applied Biosystems, CA). Quantitative PCR (qPCR) was performed using Power SYBR Green PCR Master Mix and the detection using an ABI PRISM 7500 System (Applied Biosystems). The PCR conditions were as follows: initial denaturation step at 94 °C for 5 min; 40 cycles of denaturation at 94 °C for 30 s, annealing at 60 °C for 33 s and extension at 72 °C for 30 s. At the end a final 7 minutes extension at 72 °C was performed. Primer sequences were:

| **Target gene** | **Sense primer sequence** | **Anti-sense primer sequence** | **Concentration (µM)** |
|---|---|---|---|
| GAPDH | AGCCACATCGCTCAGACACC | GTACTCAGCGCCAGCATCG | 10 |
| Rasa1 | TTCTTAGCCAGATGAATGTTG | GTCTTCCACCAATGTAGTAATC | 4 |
| EDNRA | GTGTGATGGTATGTATGGATT | AGAAGGTTGCTACAGGTT | 4 |

After amplification, the melting curve profile or agarose gel electrophoresis was used to determine the specificity of PCR products. Quantification of target genes was performed relative to the reference GAPDH (housekeeping) gene: relative expression= 2^{[-(Ct}ₛₐₘₚₗₑ^{-Ct}_{GADPH}^{)]}. The mean minimal cycle threshold values (Ct) were calculated from quadruplicate reactions.

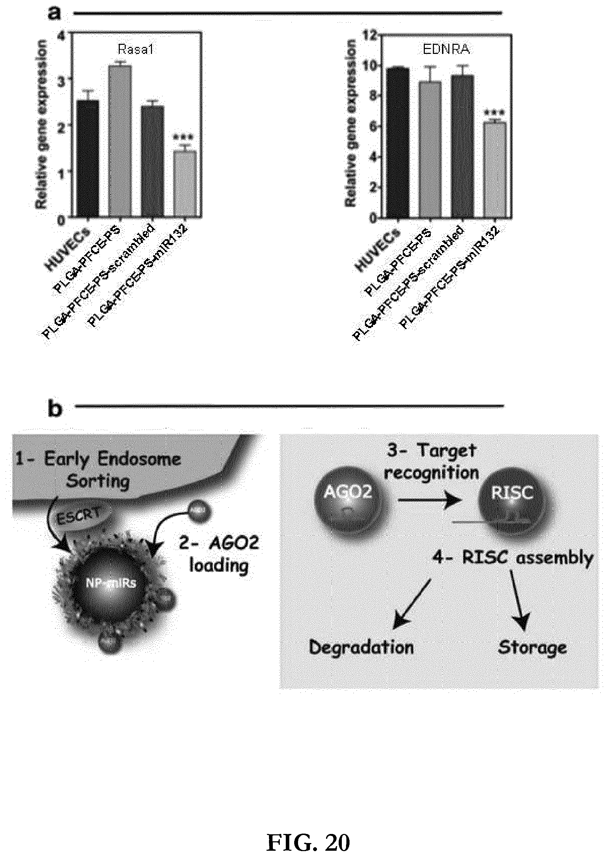

As can be seen in Figure 20(A), HUVECs transfected with PLGA-PFCE-PS-miR132 had a downregulation in the expression of Rasa1, while no effect was observed in control groups. The EDNRA results showed that the transfection of HUVECs with PLGA-PFCE-PS-miR132 also downregulated the expression of EDNRA (Figure 20(A)). In both graphs, results are expressed as an average ± SEM (*n*=4). Results were considered significant when *P* ≤ 0.001 (***).

Overall, the results showed that the PGLA-PFCE-PS formulation is able to present miRNAs very effectively to the RISC machinery, outperforming the commercial transfection system (siPORT) in terms of Ago2 assembly, as evidenced by an increased number of Ago2 foci with increasing NP-miRNA complex concentrations. It is believed that the better efficiency may be related with the intracellular route that the PGLA-PFCE-PS NPs seem to take in close association with the endomembrane system.

As illustrated in Figure 20(B) (steps 1 and 2), it is believed that PLGA-PFCE-PS-miRNA complexes may be sorted by the endosomal sorting complex required for transport (ESCRT), whereafter the miRNAs have the possibility to interact with Ago2. Since Ago proteins are located in the membrane of the endolysosomal compartment, with the miRNA binding site located in the cytosolic part of the protein, the miRNA released from the NP should cross the membrane of the endolysosomal compartment. It is conceivable that the complexation with PS might facilitate the presentation of miRNA outside the endolysosomal compartment.

Furthermore, the number of Ago foci in the cell increased with the concentration of PLGA-PFCE-PS, which shows that Ago2 is activated only in the presence of miRNA. As illustrated in steps 3 and 4 of Figure 20(B), Ago2 is required for RNA-mediated gene silencing by the RISC. In this regard, Ago2 interacts with Dicer protein during assembly of the RISC, and then is able to interact with miRNA duplex (i.e. pre-miRNA) and induce the removal of one of the miRNA strands. The Ago-miRNA complex is then ready to bind target (complementary) mRNA; Ago-miRNA complexes thus direct the RISC to mRNA targets for gene silencing. The precise mechanism of gene silencing depends on the degree of complementarity between the miRNA and its target. mRNA degradation may subsequently occur.

The close relationship between the RISC machinery and the endomembrane system may promote frequent encounters with the exogenous oligonucleotides and NPs, while the opposite happens with siPORT delivery through the diffusion and dilution of the oligonucleotides in the cytoplasm.

### Example 10: In vivo therapeutic effect of PLGA-PFCE-PS-miR132

In order to test whether PLGA-PFCE-PS-miR132 could be used to enhance endothelial cell survival and promote neovascularisation in an animal model of hindlimb ischaemia, the following procedures were followed (as approved by The Experimental Animal Committee of University of Eastern Finland).

### 10.1 Production and treatment of an animal model

Unilateral hindlimb ischaemia (UHI) was induced by ligating the right femoral artery proximal to the bifurcation of the saphenous and popliteal arteries (by ligation of both femoral artery and vein proximal to the origin of the deep femoral branch) in C57BL/6J male mice (Jackson Laboratory, Bar Harbor, ME). Prior to and after UHI the leg which received the injury was examined using VeVo Doppler to determine the perfusion levels of the femoral artery (see Example 10.3).

MILE SVEN 1 (MSi) endothelial cell line derived from mouse pancreatic islet was obtained from ATCC (CRL-2279). MS1 cells were cultured in Dulbecco's Modified Eagle's Medium (DMEM) (Sigma Aldrich) supplemented with fetal bovine serum (5%, FBS) (Biosera) and penicillin-streptomycin solution (1%, Pen/Strep) (Sigma Aldrich).

The cells were transfected with PLGA-PFCE-PS (control), PLGA-PFCE-PS-miR132 or siPORT-miR132 (as a comparison) by incubating the cells in medium (having 2% FBS) with PLGA-PFCE-PS (500 µg/ml per 400,000 MS1 cells), PLGA-PFCE-PS-miR132 (500 µg/ml per 400,000 MS1 cells, 200 nM miRNA), or siPORT-miR132 (200 nM of miRNA) for 4 h.

After transfection, the cells were washed three times with PBS, and then labeled with a IVIS probe for fluorescence detection using near-infrared lipophilic carbocyanine dye denoted by 1,1'-dioctadecyl-3,3,3',3'-tetramethylindotricarbocyanine iodide (DiR) (Kalchenko et al. (2006) J Biomed Opt 11: 050507). 1.75 µg/ml dye in PBS was used per 5×10⁶ cells, the incubation being carried out for 30 min at 37 °C, as recommended by the vendor (Caliper LifeSciences). The cells were then washed with PBS, trypsinised and centrifuged.

Fibrin gels were made by crosslinking fibrinogen in the presence of thrombin (both from Sigma-Aldrich). The fibrinogen solution was prepared by dissolving human fibrinogen in Tris-buffered saline (TBS) (Sigma-Aldrich), pH 7.4 (20 mg/ml), and then sterilized by filtering through a 0.22 µm syringe filter (Acrodisc, Pall). Fresh thrombin solutions were prepared by dissolving human thrombin in TBS at pH 7.4 at a concentration of 50 U/ml. Fibrin gels (50 µl, unless otherwise stated) were prepared by mixing three different components: fibrinogen (10 mg/ml), CaCl₂ (Merck, NJ, USA) (2.5 mM) and thrombin (2 U/ml).

The pellet obtained from centrifugation of the transfected and labeled endothelial cells was re-suspended in a fibrin gel (50 µl). The resultant solution was placed in a 1 ml diabetic syringe and kept on ice until needed for injection into the animals. For injection, 5 x10⁶ cells were prepared per syringe for injection into the gastrocnemius muscle of the operated hindlimb of each mouse. UHI mice were treated with mouse endothelial cells (MS1 cell line) alone or the transfected cells.

### 10.2 In vivo cell survival

The survival of the transplanted cells was assessed by a bioluminescence imaging (IVIS) system. This technique allows the evaluation of the spatiotemporal kinetics of endothelial cell survival.

Animals were imaged under isofluorane anaesthesia, at days 1, 3, 5 and 7 post-surgery. The transplanted cells were imaged using IVIS Lumina II hardware (Caliper LifeSciences). A laser with the values for far-red Cy5 imaging at a 640 nm excitation and 670 nm emission filter was used. Images were obtained after an exposure time of 0.5 s, using an imaging field of 12.5 × 12.5 cm.

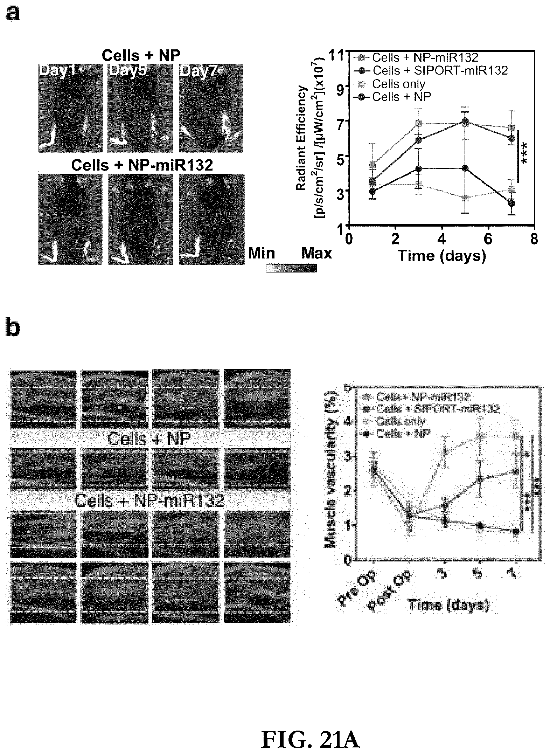

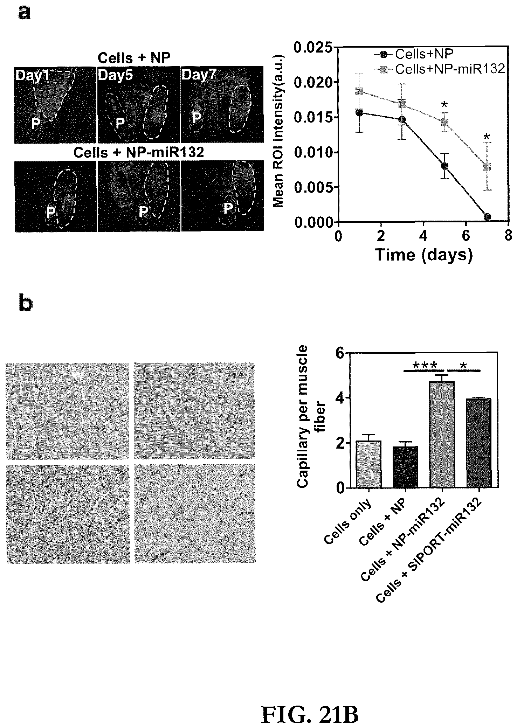

Figure 21A(a) shows representative bioluminescence images and quantitative bioluminescence intensity for endothelial-transplanted mice. As can be seen from the figure, endothelial cells transfected with PLGA-PFCE-PS-miR132 or siPORT-miR132 proliferated upon day 3, as confirmed by an increase in the radiant efficiency, and then kept the same signal until day 7. This signal was statistically higher (*P*<0.001) than animals treated with cells transfected with PLGA-PFCE-PS without miR132 or cells alone. In the latter case, the initial signal was maintained over the duration of the study and not statistically different from the one obtained at day 7.

### 10.3 In vivo blood perfusion

The IVIS results were further confirmed by the evaluation of blood perfusion using VeVo analysis.

Ischemic gastrocnemius muscle perfusion was measured on day 0 (pre- and postoperative), day 3, 5 and day 7 after surgery. Perfusion data were acquired with a high-resolution imaging system (VeVo 770, VisualSonics Inc., Toronto, ON, Canada), using an ultrasound probe (RMV-704) in Power Doppler mode (power 100%, RF-cycle 5, gain 25, velocity medium, wall filter 15, scan speed 15, priority 100, intensity range maximum 53 and minimum 19). Video clips containing approximately 50 frames were captured and the vascularity index (normalised to the area of the muscle) in three evenly separated frames was quantified with VeVo 770 measurement software (VisualSonics).

Representative images and quantification of blood perfusion analyses by VeVo are illustrated in Figure 21A(b). The results are represented as group means of ratios to intact values to reduce measurement dependent variation.

By using the VeVo technique it was possible to ensure the efficacy of the surgical procedure, as shown by a reduction of the blood perfusion in the injured limb (compare Pre OP to Post OP in Figure 21A(b)).

Over time, blood flow increased significantly (*P*<0.001) in animals treated with cells transfected with PLGA-PFCE-PS-miR132 or siPORT-miR132 relative to animals treated with cells alone or cells labeled with PLGA-PFCE-PS but without miR132 (Figure 21A(b)). This increase correlated with cell survival data attained from IVIS measurements. Importantly, animals treated with PLGA-PFCE-PS-miR132 had a statistically higher blood perfusion (*P*<0.05) than animals treated with siPORT-miR132.

### 10.4 In vivo cell location

Cell survival and location was also monitored by ¹⁹F-MRI, using a purpose built circular surface coil tuneable to ¹⁹F allowing simultaneous ¹H acquisition of anatomical leg constitution, i.e. ¹⁹F MRI was performed to detect NP-labeled cells while ¹H MRI was used to reveal the anatomy of the limbs.

MRI was performed on animals at 1, 3 5 and 7 days post-surgery. The animals were anesthetised using isofluorane (4%) in oxygen (100%). Mice were placed in a purpose built cradle for subsequent horizontal positioning in the magnet bore. Maintenance anaesthesia was 1.5-2% isofluorane at 1 l min⁻¹ oxygen flow. MRI was carried out on a 9.4T horizontal bore (60 G/cm, inner bore diameter 120 mm) system (Varian), using Direct Drive console (VJ NMR) with a linear transmit and receiver. For ¹H imaging, coronal GEMS images of the legs were taken at matrix size of 125 (later zero filled to 256), 30 averages were taken, field of view was 35 × 35 cm, 10 imaging planes each of imm depth, TE/TR = 4/1.2 ms with a flip angle of 70 degrees resulting in a total experimental time of approximately 11 minutes. The ¹H images were later isotropically zero-filled by a factor of two and filtered (modified third-order Butterworth filter) before Fourier transformation. For ¹⁹F imaging, coronal GEMS images of the legs cloned from the ¹H images were taken at matrix size of 256, 60 averages were taken, field of view was 35 × 35 cm, 1 imaging plane of 10mm depth TE/TR = 4/1.2 ms with a flip angle of 90 degrees resulting in a total experimental time of approximately 37 minutes. All images were Fourier transformed and overlaid (green showing the labeled cells with NPs by the ¹⁹F signals and grey denoting the anatomy by ¹H). At all times a phantom, denoted P, was present to allow shimming and pulse calibrations, furthermore aiding orientation within the surface coil. The intensity of the region of interest (ROI) in ¹⁹F MRI images was measured and calibrated against the internal standard.

Representative images and quantification of ¹⁹F-MRI analysis are shown in Figure 21B(a) (ROI shown as a marked zone without a label and the internal standard shown as phantom, P).

For the two experimental groups tested, i.e. animals treated with endothelial cells transfected with PLGA-PFCE-PS or PLGA-PFCE-PS-miR132, there was a decrease in the ¹⁹F signal over time (Figure 21B(a)). However, animals treated with endothelial cells transfected with PLGA-PFCE-PS-miR132 had a statistically higher ¹⁹F signal, and thus higher cell number, than animals treated with cells transfected with PLGA-PFCE-PS but without miR132 (*P*<0.05) (Figure 21B(a)).

### 10.5 Cell engraftment and neovascularisation

To further characterise cell engraftment and neovascularisation, gross anatomy and histology recordings were performed at day 7 post-operation.

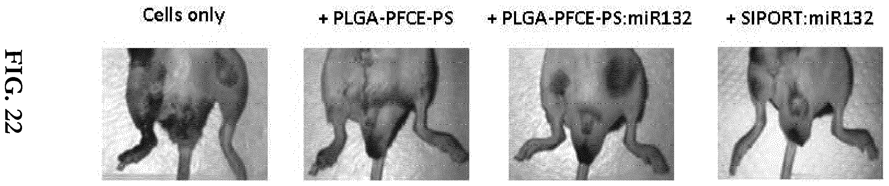

Figure 22 shows the gross anatomy images. Animals treated with endothelial cells alone or cells transfected with PLGA-PFCE-PS showed necrotic limbs and auto-amputation of toes, while animals treated with PLGA-PFCE-PS-miR132 or siPORT-miR132 had no macroscopic signs of necrosis.

The muscles of the injured limbs were further characterised by immunohistochemical staining for CD31 as a marker of endothelial cells and neovascularisation. In this regard, the animals were sacrificed (at day 7 after the operation) and perfused with PFA (1%) in citrate through the abdominal aorta. The test muscle was cut from the mouse leg and immersion fixed with 4% PFA in 7.5% sucrose for 4 h. Sections (4 µm) were immunostained with rat anti-mouse CD31- antibody (dilution 1:25, MEC 13.3; BD Biosciences Pharmingen, San Diego, CA). As a secondary antibody, biotinylated rabbit anti-rat antibody (Vector Laboratories, Burlingame, CA) was used. The avidin-biotin-horseradish peroxidise system (Vector Laboratories, CA, USA) with DAB as a chromogen (Zymed, San Francisko, California) and Tyramide signal amplification system (TBA, Biotin System, PerkinElmer, Shelton, USA) for amplification of the signal was used.

Photographs of the stained sections were taken and processed using an Olympus AX70 microscope (Olympus Optical, Tokyo, Japan) and analySIS imaging software (Soft Imaging System GmbH, Muenster, Germany), respectively. Capillary density (capillary/muscle fibre ratios) was measured from three microscopic fields of CD31 immunostained sections taken in a close proximity to the needle track at ×100 magnification in a blinded manner. Representative histologic images and quantification of capillaries per muscle fiber at 7 days postligation for the various experimental groups are shown in Figure 21B(b).

Higher levels of CD31 were observed in animals treated with endothelial cells transfected with PLGA-PFCE-PS-miR132 as compared to cells transfected with NPs but without miR (*P*<0.001) or with siPORT-miR132 (*P*<0.05) (Figure 21B(b)), which is indicative of neo-angiogenesis.

In all graphs, values are average ± SEM (*n*=5). Results were considered significant when P ≤ 0.05 (*), P ≤ 0.01 (**) and P ≤ 0.001 (***).

The significant reduction in ¹⁹F signal after 7 days for animals treated with endothelial cells transfected with PLGA-PFCE-PS (but without miRNA), combined with the IVIS, blood perfusion and immunohistochemistry analyses, suggest a poor survival and engraftment of the transplanted cells. Although animals treated with endothelial cells transfected with PLGA-PFCE-PS-miR132 had a decrease in ¹⁹F signal after 7 days (though higher than animals treated with cells without miRNA), the results obtained by the other techniques indicate that such reduction correlates with higher survival of the transplanted cells (increase in IVIS signal) and cell proliferation (increase in IVIS signal and blood perfusion). Therefore, the reduction of ¹⁹F signal is likely due to cell proliferation.

The data hence show that endothelial cells treated with PLGA-PFCE-PS-miR132 have higher survival and pro-angiogenic activity than cells without miRNA, in an ischaemic hindlimb animal model. Animals treated with cells that had been transfected with PLGA-PFCE-PS-miR132 had an increase in blood perfusion, which correlates with an increase in the near-infrared signal using IVIS and a decrease of signal in ¹⁹F-MRI, implying cell proliferation. Furthermore, the immunohistochemistry results showed an increase in the number of cells expressing the endothelial marker CD31, indicative of neo-angiogenesis. This is believed to be the first study demonstrating the therapeutic effect of a NP formulation with miR132.

### Statistical analysis

Throughout the examples described above, statistical analysis was performed as follows. For analysis involving three or more groups, ANOVA was used, followed by a Student-Newman-Keuls post hoc test. For analysis of two groups, a paired t-test was used. Statistical analysis was performed using GraphPad Prism software (San Diego, CA, USA, http://www.graphpad.com/). Results were considered significant when *P*≤ 0.05.

### Conclusions

The ability to monitor transplanted cells *in vivo* by non-invasive imaging methods such as MRI, and simultaneously manipulate them by intracellular delivery of therapeutic agents such as miRNAs, is important in the context of cell reprogramming and regenerative medicine. The Examples included herein show, for the first time, that biodegradable NPs, containing an imaging agent such as PFCE, can track cells *in vivo* and release their payload efficiently in endothelial cells. The data also show that intracellular release of the therapeutic agent from the NPs is associated with an *in vivo* therapeutic effect.

Thus, as the data show, NP formulations as described herein permit, simultaneously, the efficient delivery to target cells of a therapeutic agent and the tracking of cells, not only *in vitro,* but also non-invasively *in vivo,* by MRI. MRI is the most attractive imaging modality to track cells because it provides high quality three-dimensional functional and anatomic information with high contrast. As the data show, MRI can readily monitor endothelial cells transfected with PLGA-PFCE.

In contrast to known fluorine-based formulations that have previously been used to track cells *in vivo,* encapsulating the fluorine-based compound in PLGA, as described herein, is advantageous, as it enables the provision of NPs that have a defined size, are relatively stable and can be easily functionalised.

The data also show that NP-miRNA formulations as described herein are able to interact with Ago2 and GW182 proteins, molecular players involved in the biological action of miRNAs. Moreover, the release of miRNA by the NPs was proven to promote survival of endothelial cells transplanted *in vivo,* and contribute to vascularisation and blood perfusion in ischaemic limbs.
Although the data were obtained using NP-miRNA formulations having therapeutic efficacy in the treatment of ischaemia, they are proof of concept for the delivery of miRNAs having therapeutic efficacy in the treatment of other conditions, diseases and disorders, as identified herein, and indeed for the delivery of other therapeutic agents entirely. It will be within the remit of the skilled person to adapt the particular formulations and methods exemplified herein as necessary, in order to repeat the invention using other therapeutic agents, and in other cell types, to treat other diseases, disorders and conditions as described herein.

Thus, many modifications and variations to the described examples are possible. For example, any of the NPs described herein can be customised in terms of content (polymer, therapeutic agent, imaging agent), coating, surface charge and size. The NPs are also adaptable to any imaging method, notably MRI, fluorescence imaging and confocal microscopy.

## Claims

1. A nanoparticle conjugate for intracellular delivery of a therapeutic agent, wherein the conjugate comprises a nanoparticle, a cationic agent, the therapeutic agent and at least one imaging agent including a perfluorocarbon, wherein the cationic agent is protamine sulphate.

2. A nanoparticle conjugate as claimed in claim 1, wherein:
a) the intracellular delivery is mediated by endosomes, optionally wherein the nanoparticles are retained in the endolysosomal compartment;
b) the nanoparticle is polymeric, optionally wherein the nanoparticle is formed from poly(lactic acid-co-glycolic acid);
c) the at least one imaging agent is selected from the group consisting of perfluorocarbons and fluorescent labels;
d) the perfluorocarbon is perfluoro-1,5-crown ether; and/or
e) the therapeutic agent is a protein, peptide or oligonucleotide, optionally wherein the oligonucleotide is miRNA.

3. A nanoparticle conjugate as claimed in claim 1 or claim 2, comprising poly(lactic acid-co-glycolic acid), perfluoro-1,5-crown ether, protamine sulphate and miRNA.

4. A nanoparticle conjugate for use in a method of transfecting a mammalian cell with a therapeutic oligonucleotide,
the method comprising contacting the mammalian cell with the conjugate,
and the conjugate comprising a nanoparticle, a cationic agent, the therapeutic oligonucleotide and at least one imaging agent including a perfluorocarbon, wherein the cationic agent is protamine sulphate.

5. A nanoparticle conjugate as claimed in claim 4, wherein the method is an in *vitro, ex vivo* or *in vivo* method.

6. A mammalian cell transfected with a nanoparticle conjugate, the conjugate comprising a nanoparticle, a cationic agent, a therapeutic oligonucleotide and at least one imaging agent including a perfluorocarbon, wherein the cationic agent is protamine sulphate.

7. A nanoparticle conjugate as claimed in claim 4 or claim 5, or a mammalian cell as claimed in claim 6, wherein the mammalian cell is an endothelial cell, a mononuclear cell, a cardiac progenitor cell or a bone marrow cell.

8. A pharmaceutical composition comprising a nanoparticle conjugate as claimed in any one of claims 1-3 and/or a mammalian cell as claimed in claim 6 or claim 7.

9. A kit comprising a nanoparticle conjugate as claimed in any one of claims 1-3 and a mammalian cell, wherein the therapeutic agent comprised in the conjugate is an oligonucleotide and the mammalian cell is suitable for transfection with the nanoparticle conjugate.

10. A nanoparticle conjugate for use in therapy, wherein the conjugate comprises a nanoparticle, a cationic agent, a therapeutic agent and at least one imaging agent including a perfluorocarbon, wherein the cationic agent is protamine sulphate.

11. A mammalian cell, transfected with a nanoparticle conjugate, for use in therapy, the conjugate comprising a nanoparticle, a cationic agent, a therapeutic oligonucleotide and at least one imaging agent including a perfluorocarbon, wherein the cationic agent is protamine sulphate.

12. A nanoparticle conjugate as claimed in any one of claims 4, 5, 7 or 10, a mammalian cell as claimed in any one of claims 6, 7 or 11, or a pharmaceutical composition as claimed in claim 8, wherein the nanoparticle conjugate is as claimed in any one of claims 1-3.

13. A nanoparticle conjugate, mammalian cell or pharmaceutical composition for use in a method of inducing or preventing angiogenesis in a subject or in a tissue, the method comprising administering the nanoparticle conjugate, mammalian cell or pharmaceutical composition to the subject or said tissue, wherein:
a) the nanoparticle conjugate is as claimed in any one of claims 1-3 or 10;
b) the mammalian cell is as claimed in any one of claims 6, 11 or 12; and
c) the pharmaceutical composition is as claimed in claim 8 or claim 12.

14. A nanoparticle conjugate, mammalian cell or pharmaceutical composition for use in a method of treating, ameliorating or preventing a myocardial infarction, an ischaemic disease, disorder or condition or cancer in a subject, the method comprising administering the nanoparticle conjugate, mammalian cell or pharmaceutical composition to the subject, wherein:
a) the nanoparticle conjugate is as claimed in any one of claims 1-3 or 10;
b) the mammalian cell is as claimed in any one of claims 6, 11 or 12; and
c) the pharmaceutical composition is as claimed in claim 8 or claim 12.

15. A delivery system for use in increasing or decreasing blood flow in *vivo* or in tissues *in vitro* or *ex vivo,* the delivery system comprising a nanoparticle conjugate as claimed in any one of claims 1-3 or 10 or a mammalian cell as claimed in any one of claims 6, 7 or 11.

## Patentansprüche

1. Nanoteilchen-Konjugat zur intrazellulären Verabreichung eines Therapeutikums, wobei das Konjugat ein Nanoteilchen, eine kationische Substanz, das Therapeutikum und wenigstens eine bildgebende Substanz umfasst, die Perfluorkohlenstoff aufweist, wobei die kationische Substanz Protaminsulfat ist.

2. Nanoteilchen-Konjugat nach Anspruch 1, wobei:
a) die intrazelluläre Verabreichung durch Endosomen vermittelt wird, wobei die Nanoteilchen optional in dem Endosomen-Kompartiment gehalten werden;
b) das Nanoteilchen polymer ist, wobei das Nanoteilchen optional aus Poly(milchsäure-co-glycolsäure) gebildet wird;
c) die wenigstens eine bildgebende Substanz ausgewählt ist aus der Gruppe bestehend aus Perfluorkohlenstoffen und Fluoreszenzmarkierungen;
d) der Perfluorkohlenstoff Perfluor-1,5-Kronenether ist; und/oder
e) das Therapeutikum ein Protein, Peptid oder Oligonucleotid ist, wobei das Oligonucleotid optional miRNA ist.

3. Nanoteilchen-Konjugat nach Anspruch 1 oder 2, umfassend Poly(milchsäure-co-glycolsäure), Perfluor-1,5-Kronenether, Protaminsulfat und miRNA.

4. Nanoteilchen-Konjugat zur Verwendung in einem Verfahren zum Transfizieren einer Säugetierzelle mit einem therapeutischen Oligonucleotid,
wobei das Verfahren das Inkontaktbringen der Säugetierzelle mit dem Konjugat umfasst;
und wobei das Konjugat ein Nanoteilchen, eine kationische Substanz, das therapeutische Oligonucleotid und wenigstens eine bildgebende Substanz umfasst, die Perfluorkohlenstoff aufweist, wobei die kationische Substanz Protaminsulfat ist.

5. Nanoteilchen-Konjugat nach Anspruch 4, wobei das Verfahren ein *in vitro-, ex vivo-* oder *in vivo*-Verfahren ist.

6. Säugetierzelle, die mit einem Nanoteilchen-Konjugat transfiziert ist, wobei das Konjugat ein Nanoteilchen, eine kationische Substanz, ein therapeutisches Oligonucleotid und wenigstens eine bildgebende Substanz umfasst, die Perfluorkohlenstoff aufweist, wobei die kationische Substanz Protaminsulfat ist.

7. Nanoteilchen-Konjugat nach Anspruch 4 oder 5 oder eine Säugetierzelle nach Anspruch 6, wobei die Säugetierzelle eine Endothelzelle, eine mononukleäre Zelle, eine Herz-Vorläuferzelle oder eine Knochenmarkzelle ist.

8. Pharmazeutische Zusammensetzung, umfassend ein Nanoteilchen-Konjugat nach einem der Ansprüche 1 bis 3 und/oder eine Säugetierzelle nach Anspruch 6 oder 7.

9. Kit, umfassend ein Nanoteilchen-Konjugat nach einem der Ansprüche 1 bis 3 und eine Säugetierzelle, wobei das Therapeutikum in dem Konjugat ein Oligonucleotid ist, und wobei sich die Säugetierzelle für eine Transfektion mit dem Nanoteilchen-Konjugat eignet.

10. Nanoteilchen-Konjugat zur Verwendung in der Behandlung, wobei das Konjugat ein Nanoteilchen, eine kationische Substanz, ein Therapeutikum und wenigstens eine bildgebende Substanz umfasst, die Perfluorkohlenstoff aufweist, wobei die kationische Substanz Protaminsulfat ist.

11. Säugetierzelle, die mit einem Nanoteilchen-Konjugat transfiziert ist, zur Verwendung in der Behandlung, wobei das Konjugat ein Nanoteilchen, eine kationische Substanz, ein therapeutisches Oligonucleotid und wenigstens eine bildgebende Substanz umfasst, die Perfluorkohlenstoff aufweist, wobei die kationische Substanz Protaminsulfat ist.

12. Nanoteilchen-Konjugat nach einem der Ansprüche 4, 5, 7 oder 10, eine Säugetierzelle nach einem der Ansprüche 6, 7 oder 11 oder eine pharmazeutische Zusammensetzung nach Anspruch 8, wobei das Nanoteilchen-Konjugat einem der Ansprüche 1 bis 3 entspricht.

13. Nanoteilchen-Konjugat, Säugetierzelle oder pharmazeutische Zusammensetzung zur Verwendung in einem Verfahren zur Induzierung oder Prävention von Angiogenese in einem Subjekt oder in einem Gewebe, wobei das Verfahren das Verabreichen des Nanoteilchen-Konjugats, der Säugetierzelle oder der pharmazeutischen Zusammensetzung an das Subjekt oder das Gewebe umfasst, wobei:
a) das Nanoteilchen-Konjugat einem der Ansprüche 1 bis 3 oder 10 entspricht;
b) die Säugetierzelle einem der Ansprüche 6, 11 oder 12 entspricht; und
c) die pharmazeutische Zusammensetzung Anspruch 8 oder 12 entspricht.

14. Nanoteilchen-Konjugat, Säugetierzelle oder pharmazeutische Zusammensetzung zur Verwendung in einem Verfahren zur Behandlung, Besserung oder Prävention eines Herzinfarkts, einer ischämischen Erkrankung, Störung oder eines solchen Zustands oder von Krebs in einem Subjekt, wobei das Verfahren das Verabreichen des Nanoteilchen-Konjugats, der Säugetierzelle oder der pharmazeutischen Zusammensetzung an das Subjekt umfasst, wobei:
a) das Nanoteilchen-Konjugat einem der Ansprüche 1 bis 3 oder 10 entspricht;
b) die Säugetierzelle einem der Ansprüche 6, 11 oder 12 entspricht; und
c) die pharmazeutische Zusammensetzung Anspruch 8 oder 12 entspricht.

15. Verabreichungssystem zur Verwendung in der Steigerung oder Verringerung der Blutströmung *in vivo* oder in Geweben *in vitro* oder *ex vivo,* wobei das Verabreichungssystem ein Nanoteilchen-Konjugat nach einem der Ansprüche 1 bis 3 oder 10 oder eine Säugetierzelle nach einem der Ansprüche 6, 7 oder 11 umfasst.

## Revendications

1. Conjugué de nanoparticule pour administration intracellulaire d'un agent thérapeutique, dans lequel le conjugué comprend une nanoparticule, un agent cationique, l'agent thérapeutique et au moins un agent d'imagerie comprenant un perfluorocarbone, dans lequel l'agent cationique est le sulfate de protamine.

2. Conjugué de nanoparticule selon la revendication 1, dans lequel :
a) l'administration intracellulaire est médiée par des endosomes, éventuellement dans lequel les nanoparticules sont retenues dans le compartiment endolysosomal ;
b) la nanoparticule est polymérique, éventuellement dans lequel la nanoparticule est formée à partir d'un poly(acide lactique-acide co-glycolique) ;
c) le au moins un agent d'imagerie est choisi parmi le groupe constitué de perfluorocarbones et d'étiquettes fluorescentes ;
d) le perfluorocarbone est un perfluoro-1,5-éther couronne ; et/ou
e) l'agent thérapeutique est une protéine, un peptide ou un oligonucléotide, éventuellement dans lequel l'oligonucléotide est un miARN.

3. Conjugué de nanoparticule selon la revendication 1 ou la revendication 2, comprenant un poly(acide lactique-acide co-glycolique), le perfluoro-1,5-éther couronne, le sulfate de protamine et un miARN.

4. Conjugué de nanoparticule pour utilisation dans un procédé de transfection d'une cellule mammalienne avec un oligonucléotide thérapeutique, le procédé comprenant la mise en contact de la cellule mammalienne avec le conjugué, et le conjugué comprenant une nanoparticule, un agent cationique, l'oligonucléotide thérapeutique et au moins un agent d'imagerie comprenant un perfluorocarbone, dans lequel l'agent cationique est le sulfate de protamine.

5. Conjugué de nanoparticule selon la revendication 4, dans lequel le procédé est un procédé in vitro, ex vivo ou in vivo.

6. Cellule mammalienne transfectée avec un conjugué de nanoparticule, le conjugué comprenant une nanoparticule, un agent cationique, un oligonucléotide thérapeutique et au moins un agent d'imagerie comprenant un perfluorocarbone, dans lequel l'agent cationique est le sulfate de protamine.

7. Conjugué de nanoparticule selon la revendication 4 ou la revendication 5, ou une cellule mammalienne selon la revendication 6, dans lequel la cellule mammalienne est une cellule endothéliale, une cellule mononuclée, une cellule progénitrice cardiaque ou une cellule de moelle osseuse.

8. Composition pharmaceutique comprenant un conjugué de nanoparticule selon l'une quelconque des revendications 1 à 3 et/ou une cellule mammalienne selon la revendication 6 ou la revendication 7.

9. Trousse comprenant un conjugué de nanoparticule selon l'une quelconque des revendications 1 à 3 et une cellule mammalienne, dans laquelle l'agent thérapeutique compris dans le conjugué est un oligonucléotide et la cellule mammalienne est appropriée pour transfection avec le conjugué de nanoparticule.

10. Conjugué de nanoparticule pour utilisation dans une thérapie, dans lequel le conjugué comprend une nanoparticule, un agent cationique, un agent thérapeutique et au moins un agent d'imagerie comprenant un perfluorocarbone, dans lequel l'agent cationique est le sulfate de protamine.

11. Cellule mammalienne, transfectée avec un conjugué de nanoparticule, pour utilisation dans une thérapie, le conjugué comprenant une nanoparticule, un agent cationique, un oligonucléotide thérapeutique et au moins un agent d'imagerie comprenant un perfluorocarbone, dans lequel l'agent cationique est le sulfate de protamine.

12. Conjugué de nanoparticule selon l'une quelconque des revendications 4, 5, 7 ou 10, une cellule mammalienne selon l'une quelconque des revendications 6, 7, ou 11, ou une composition pharmaceutique selon la revendication 8, dans lequel le conjugué de nanoparticule est selon l'une quelconque des revendications 1 à 3.

13. Conjugué de nanoparticule, cellule mammalienne ou composition pharmaceutique pour utilisation dans un procédé d'induction ou de prévention d'une angiogenèse chez un sujet ou dans un tissu, le procédé comprenant l'administration du conjugué de nanoparticule, de la cellule mammalienne ou de la composition pharmaceutique au sujet ou audit tissu, dans lequel :
a) le conjugué de nanoparticule est selon l'une quelconque des revendications 1 à 3 ou 10 ;
b) la cellule mammalienne est selon l'une quelconque des revendications 6, 11 ou 12 ; et
c) la composition pharmaceutique est selon la revendication 8 ou la revendication 12.

14. Conjugué de nanoparticule, cellule mammalienne ou composition pharmaceutique pour utilisation dans un procédé de traitement, d'amélioration ou de prévention d'un infarctus du myocarde, d'une maladie, d'un trouble ou d'une condition ischémique ou d'un cancer chez un sujet, le procédé comprenant l'administration du conjugué de nanoparticule, de la cellule mammalienne ou de la composition pharmaceutique au sujet, dans lequel :
a) le conjugué de nanoparticule est selon l'une quelconque des revendications 1 à 3 ou 10 ;
b) la cellule mammalienne est selon l'une quelconque des revendications 6, 11 ou 12 ; et
c) la composition pharmaceutique est selon la revendication 8 ou la revendication 12.

15. Système d'administration pour utilisation dans un flux sanguin croissant ou décroissant in vivo ou dans des tissus in vitro ou ex vivo, le système d'administration comprenant un conjugué de nanoparticule selon l'une quelconque des revendications 1 à 3 ou 10, ou une cellule mammalienne selon l'une quelconque des revendications 6, 7 ou 11.


### SEQUENCE LISTING

<110> BIOCANT ASSOCIAÇÃO DE TRANSFERÊNCIA DE TECNOLOGIA
<120> NANOPARTICLES AND USES THEREOF
<130> TH/65079PCT1
<140> PCT/EP2012/065550
   <141> 2012-08-08
<150> EP11398008.0
   <151> 2011-12-16
<160> 6
<170> PatentIn ver. 3.5
<210> 1
   <211> 20
   <212> DNA
   <213> Artifical Sequence
<220>
   <223> Primer
<400> 1
   AGCCACATCGCTCAGACACC
<210> 2
   <211> 19
   <212> DNA
   <213> Artifical Sequence
<220>
   <223> Primer
<400> 2
   GTACTCAGCGCCAGCATCG
<210> 3
   <211> 21
   <212> DNA
   <213> Artifical Sequence
<220>
   <223> Primer
<400> 3
   TTCTTAGCCAGATGAATGTTG
<210> 4
   <211> 22
   <212> DNA
   <213> Artifical Sequence
<220>
   <223> Primer
<400> 4
   GTCTTCCACCAATGTAGTAATC
<210> 5
   <211> 21
   <212> DNA
   <213> Artifical Sequence
<220>
   <223> Primer
<400> 5
   GTGTGATGGTATGTATGGATT
<210> 6
   <211> 18
   <212> DNA
   <213> Artifical Sequence
<220>
   <223> Primer
<400> 6
   AGAAGGTTGCTACAGGTT